# EUROPÄISCHE PATENTSCHRIFT

(11) **EP 2 729 484 B1**
(45) Veröffentlichungstag und Bekanntmachung des Hinweises auf die Patenterteilung: **29.07.2015**
(21) Anmeldenummer: 12738069.9
(22) Anmeldetag: 09.07.2012
(51) Int. Cl.: C07K 5/065, A61K 38/06, A61K 38/12, A61P 7/02

(54) **ZYKLISCHE TRIPEPTIDMIMETIKA ALS PLASMIN INHIBITOREN**
CYCLIC TRIPEPTIDE MIMETICS AS PLASMIN INHIBITORS
TRIPEPTIDOMIMÉTIQUES CYCLIQUES UTILISÉS EN TANT QU'INHIBITEURS DE PLASMINE

(30) Priorität: 07.07.2011 EP 11173132
(43) Veröffentlichungstag der Anmeldung: 14.05.2014
(73) Patentinhaber: Philipps Universität Marburg, 35037 Marburg (DE)
(72) Erfinder: SAUPE, Sebastian, Martin, 36452 Kaltennordheim (DE); STEINMETZER, Torsten, 07743 Jena (DE)
(74) Vertreter: Patentanwälte Olbricht Buchhold Keulertz
(86) Internationale Anmeldenummer: PCT/EP2012/063363
(87) Internationale Veröffentlichungsnummer: WO 2013/004845

(56) Entgegenhaltungen:
- WO-A1-2008/049595
- WO-A2-2012/004678

## Beschreibung

Die Erfindung betrifft N-terminal modifizierte zyklische Tripeptidmimetika, die als Hemmstoffe der Serinprotease Plasmin geeignet sind, die Fibrinolyse zu hemmen und dadurch den Blutverlust bei Operationen zu mindern.

### Einleitung und Stand der Technik

Plasmin ist eine trypsinartige Serinprotease, die Peptid- und Proteinsubstrate C-terminal der basischen Aminosäuren Arginin oder Lysin spaltet. Plasmin wird aus dem Zymogen Plasminogen durch katalytische Wirkung der Plasminogenaktivatoren Urokinase oder tPA gebildet. Zu den Plasminsubstraten gehören verschiedene Proteine der extrazellulären Matrix und Basalmembran, beispielsweise Fibronectin, Laminin, Type IV-Kollagen oder Fibrin, aber auch zahlreiche Zymogene, wie Proformen der Matrix-Metalloproteasen oder des Plasminogenaktivators Urokinase. Im Blut ist Plasmin vor allem für die Fibrinolyse verantwortlich, indem es Fibrin in lösliche Produkte spaltet.

Unter bestimmten pathologischen Bedingungen kann es zu einer spontanen Aktivierung der Fibrinolyse kommen. Im Falle einer derartigen Hyperplasminämie wird nicht nur das wundverschließende Fibrin abgebaut, sondern es werden auch gerinnungshemmende Fibrinogenabbauprodukte gebildet. Dadurch können schwere Blutstillungsstörungen auftreten.

Wahrscheinlich spielt Plasmin auch eine wichtige Rolle bei der Rheumatoiden Arthritis, indem es beim Abbau von Gelenksknorpel und Matrixproteinen beteiligt ist (Li et al., Am. J. Pathology 2005, 166, 783-792). Plasmin ist auch als ein wichtiger Aktivator zahlreicher inaktiver Proformen von Proteasen und von Angiogenese- oder anderen Wachstumsfaktoren beschrieben, die die Tumorbildung und Metastasierung fördern. Beschrieben wurde auch eine Funktion des Plasmins bei der proliferativen Vitreoretinopathie (PVR) über eine plasminkatalysierte Aktivierung des PDFG-C (platelet-derived growth factor C, Lei et al., Investigative Ophthalmology & Visual Science, January 2008, 49, 42-48). Die PVR kann zu Netzhautablösungen im Auge führen und ist eine der hauptsächlichen Komplikation bei Operationen an der Netzhaut im Auge. Inzwischen gibt es auch mehrere klinische Studien zur Verwendung des Plasmins zur Thrombolysetherapie nach Herzinfarkten (Marder et al., Stroke. 2010, 41, Suppl. 1 S45-S49). Wie bei der Thrombolysetherapie mit Plasminogenaktivatoren (Urokinase, tPA, Streptokinase) kann es auch bei der Verwendung des Plasmins zu Blutungskomplikationen kommen, die ein geeignetes Antidot erfordern, beispielsweise einen effizienten Plasmininhibitor.

Als Antifibrinolytika werden in der Klinik synthetische Aminocarbonsäuren, wie ε-Aminocapronsäure, p-Aminomethylbenzoesäure oder Tranexaminsäure (trans-4-Aminomethylcyclohexancarbonsäure, nach neuer Nomenklatur Tranexamsäure) verwendet. Diese Verbindungen blockieren die Bindung des Zymogens Plasminogen am Fibrin und hemmen dadurch dessen Aktivierung zu Plasmin. Deshalb sind diese Verbindungen keine direkten Hemmstoffe des Plasmins und können bereits gebildetes Plasmin nicht inhibieren. Oral verabreichte Tranexamsäure (Lysteda^{®}) wird in den USA auch für die Behandlung starker Regelblutungen eingesetzt. Diese Verbindungen können auch zur Verhinderung oder Linderung von Blutungen bei Zahnextraktion oder Zahnfleischblutungen verwendet werden, vor allem bei Patienten mit Hämophilie. Prinzipiell können alle Wirkstoffe, die entweder Plasmin direkt hemmen oder dessen Bildung inhibieren, auch zur Therapie leichter Formen der Hämophilie eingesetzt werden.

Als weiteres Antifibrinolytikum wurde Aprotinin (Trasylol^{®}) eingesetzt, ein Polypeptid aus 58 Aminosäuren, das aus Rinderlunge gewonnen wird. Aprotinin inhibiert Plasmin mit einer Hemmkonstante von 1 nM, ist aber relativ unspezifisch und hemmt beispielsweise auch wirksam Trypsin (kᵢ = 0,1 nM) und Plasmakallikrein
(Kᵢ = 30 nM). Eine Hauptanwendung des Aprotinins diente der Reduktion von Blutverlust, insbesondere bei herzchirurgischen Eingriffen mit cardiopulmonalem Bypass (CPB), wodurch der Bedarf an perioperativen Bluttransfusionen deutlich reduziert wurde (Sodha et al., Expert Rev.- Cardiovasc. Ther. 2006, 4, 151-160). Aprotinin wird auch als Zusatz in Fibrinklebern eingesetzt. Die Verwendung von Aprotinin hat mehrere Nachteile. Da es aus Rinderorganen isoliert wird, besteht prinzipiell die Gefahr von pathogenen Kontaminationen und allergischen Reaktionen. Das Risiko eines anaphylaktischen Schocks ist bei erstmaliger Gabe von Aprotinin relativ gering (< 0,1 %), erhöht sich bei wiederholter Gabe innerhalb von 200 Tagen allerdings auf 4-5 %. Vor einiger Zeit wurde berichtet, dass die Gabe von Aprotinin im direkten Vergleich zu ε-Aminocapronsäure oder Tranexaminsäure eine erhöhte Anzahl an Nebenwirkungen induziert (Mangano et al., New Engl. J. Med. 2006, 354, 353-365). Durch die Gabe von Aprotinin kam es zu einer Verdopplung der Anzahl von Nierenschädigungen, wodurch eine Dialyse erforderlich wurde. Ebenso erhöhte sich durch die Gabe von Aprotinin das Herzinfarkt- und Hirnschlagrisiko im Vergleich zu den Kontrollgruppen. Dies führte dazu, dass Aprotinin im Jahr 2008 weitestgehend vom Markt genommen wurde.

Bisher wurden erst relativ wenige synthetische Plasmininhibitoren entwickelt. Von Sanders und Seto (J. Med. Chem. 1999, 42, 2969-2976) wurden schwach wirksame 4-Heterocyclohexanon-Derivate mit Hemmkonstanten ≥ 50 µM für Plasmin beschrieben. Xue und Seto berichten von peptidischen Cyclohexanon-Derivaten mit IC₅₀-Werten ≥ 2 µM (J. Med. Chem. 2005, 48, 6908-6917). Von Okada und Tsuda wurden Derivate mit einem 4-Aminomethylcyclohexanoyl-Rest hergestellt, die Plasmin mit IC₅₀-Werten ≥ 0,1 µM inhibieren (Okada et al., Chem. Pharm. Bull. 2000, 48, 1964-1972; Tsuda et al., Chem. Pharm. Bull. 2001, 49, 1457-1463). Wirksame Plasminhemmstoffe wurden kürzlich in WO 2008/049595 und von Dietrich et al. (Anesthesiolohy 2009, 110, 123-130) beschrieben, allerdings sind einige der genannten Derivate auch Hemmstoffe anderer trypsinartiger Serinproteasen., beispielsweise der Gerinnungsfaktoren Faktor Xa, Thrombin oder des Proteins Ca.
Im Falle von Selektivitätsuntersuchungen bei der Entwicklung von Thrombin- oder Faktor Xa-Inhibitoren wurden für einige Derivate auch Hemmkostanten für Plasmin angegeben. So hemmt beispielsweise der Thrombininhibitor Melagatran Plasmin mit einem Kᵢ-Wert von 0,7 µM (Gustafsson et al., Thromb. Haemost. 1998, 79, 110-118), während die strukurell eng verwandte Verbindungen H317/86 eine Hemmkonstante von 0,22 µM besitzt. Beide Verbindungen hemmen allerdings die Protease Thrombin deutlich stärker mit Kᵢ-Werten ≤ 2 nM.

Wie einleitend beschrieben gehört Plasmin mit ca. 70 weiteren Vertretern zur Gruppe der trypsinartigen Serinproteasen. Die große Anzahl strukturell ähnlicher Proteasen erschwert die Entwicklung selektiver substratanaloger Inhibitoren. Viele dieser Serinproteasen besitzen charakteristisch ausgebildete Taschen, die für die spezifische Bindung bestimmter Aminosäurereste von Substraten verantwortlich sind und zur Affinität substratanaloger Inhibitoren beitragen. Bei vielen trypsinartigen Serinproteasen beeinflusst die Aminosäure in Position 99 (Nummerierung basiert auf der Sequenz des Chymotrypsinogens) die Größe der S2-Tasche (Definition der Bindetaschen in Anlehnung an Schechter und Berger, Biochem. Biophys. Res. Comm. 27, 157-162) und grenzt sie auch von anderen Bindetaschen ab. Im Falle des Plasmins fehlt der Rest 99 und einige angrenzende Aminosäuren sogar komplett. Dies führt dazu, dass das aktive Zentrum des Plasmins relativ offen und für viele Substrate frei zugänglich ist. Deshalb hat Plasmin wahrscheinlich auch eine geringe Substratspezifität und spaltet viele verschiedene Substrate.

### Aufgabe

Der Erfindung liegt die Aufgabe zu Grunde, für therapeutische Anwendungen geeignete synthetische Wirkstoffe bereitzustellen, die in Analogie zum Aprotinin Plasmin wirksam inhibieren und daher zur Blutstillung bei verschieden Anwendungen geeignet sind, beispielsweise bei chirurgischen Eingriffen, speziell bei herzchirurgischen Eingriffen mit CPB oder bei Transplantationen. Diese Verbindungen können auch zur Behandlung starker Regelblutungen und zur Verhinderung oder Linderung von Blutungen bei Zahnextraktion oder Zahnfleischblutungen eingesetzt werden, vor allem bei Patienten mit Hämophilie. Prinzipiell können alle Wirkstoffe, die entweder Plasmin direkt hemmen auch zur Therapie leichter Formen der Hämophilie eingesetzt werden oder zur Behandlung von Krebs und Metastasierung, wenn Plasmin an diesen pathophysiologischen Prozessen beteiligt ist.

### Lösung der Aufgabe

Wir haben überraschend gefunden, dass man mit zyklisierten Tripeptidmimetika gemäß der allgemeinen Formel (I) 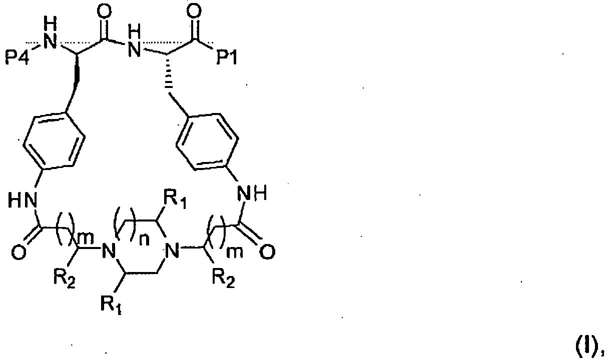 gut lösliche, wirksame und selektive Plasminhemmstoffe erhält. Die Ringstruktur wird durch Acylierung mittels eines geeigneten, substituierten Piperazin-Linkerderivates zwischen den Seitenketten des d-4-Aminophenylalanins in P3-Position und des 4-Aminophenylalanins in P2-Position substratanaloger Inhibitoren trypsinartiger Serinproteasen erhalten (Definition der P2- und P3-Aminosäuren in Anlehnung an Schechter und Berger, Biochem. Biophys. Res. Comm. 27, 157-162).

Die Verbindungen der allgemeinen Formel (I) können auch als ein Salz dieser Verbindung vorliegen, dadurch gekennzeichnet, dass
- n gleich 1 oder 2 ist, und
- m gleich 0, 1 oder 2 ist, und
- R₁ entweder H oder ein verzweigtes oder unverzweigtes Alkyl mit bis zu 3 Kohlenstoffatomen ist, und
- R₂ entweder H oder ein verzweigtes oder unverzweigtes Alkyl mit bis zu 5 Kohlenstoffatomen oder ein Aryl- bzw. Aralkylrest mit bis zu 7 Kohlenstoffatomen ist, und
- P1 einer der folgenden Reste 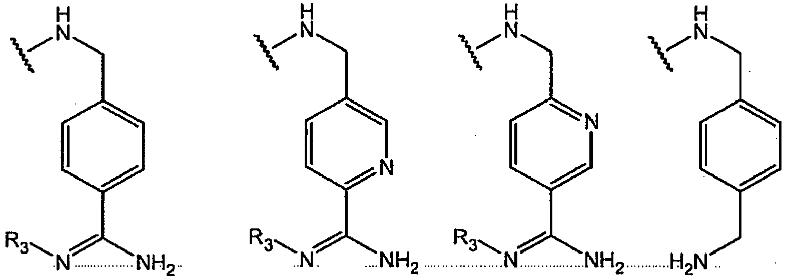 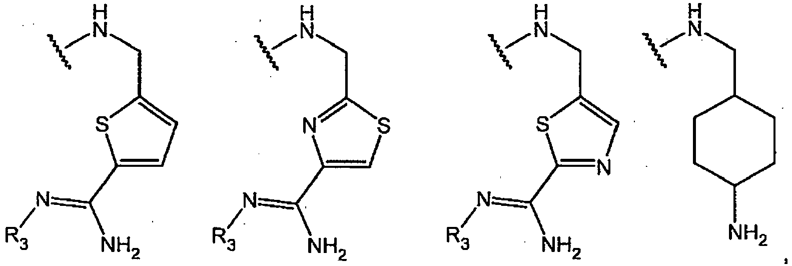 ist, wobei R₃ ein H, OH, O-CH₃, NH₂, O-CO-CH₃ oder CO-O-(CH₂)_{z}-CH₃ ist und z eine ganze Zahl von 1 bis 5, und
- P4 entweder ein H, SO₂-R₄, SO₂-NH₂, SO₂-NH-R₄, SO₂-N(R₄)₂, CO-O-R₄, CO-R₄, CH₂-COOH oder ein CH₂-COOEt ist, wobei R₄ ein verzweigter oder unverzweigter oder zyklischer Alkylrest mit 1 bis 10 Kohlenstoffatomen, oder ein Aryl-, ein Heteroaralkyl- oder Aralkylrest mit 6 bis 10 Kohlenstoffatomen sein kann, wobei der Heteroaralkylrest 1 bis 3 Heteroatome ausgewählt aus N, S oder O enthalten kann, und wobei der genannte Alkyl-, Aryl-, Aralkyl- und Heteroaralkylrest gegebenenfalls mit 1 bis 2 Resten an beliebiger Stelle substituiert sein kann, die ausgewählt werden aus Aminomethylen, Cyano, CF₃, Tetrazol, F, Cl, Br, COOH, COOEt, COOMe, Methoxy, Ethoxy, Isopropoxy, Methyl, Ethyl, oder Isopropyl ist,
   und
   wobei die Verbindung gemäß Formel (I) nicht 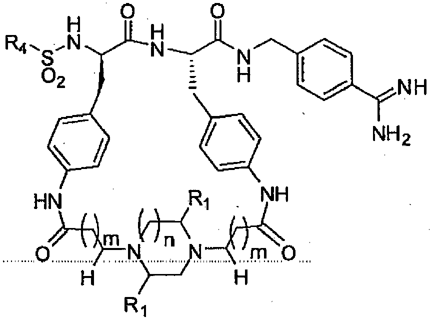 mit R₁, R₄, m und n wie oben definiert ist.

Bevorzugte Verbindungen der allgemeinen Formel (I) sind dadurch charakterisiert, dass R₁ ein H ist.

Weitere bevorzugte Verbindungen der allgemeinen Formel (I) sind dadurch gekennzeichnet, dass R₂ ein H ist.

Weitere bevorzugte Verbindungen der allgemeinen Formel (I) sind dadurch gekennzeichnet, dass P4 ein H, SO₂-NH₂ oder eine der folgenden Strukturen 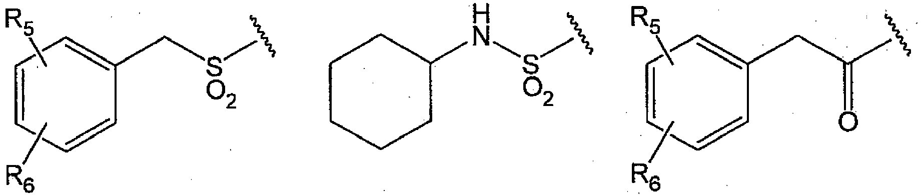 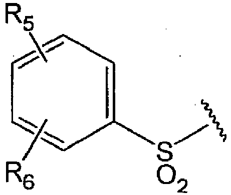 ist, wobei R₅ und R₆ unabhängig voneinander entweder H oder Aminomethylen, Cyano, CF₃, Tetrazol, F, Cl, Br, COOH, COOEt, COOMe, Methoxy, Ethoxy, Isopropoxy, Methyl, Ethyl, oder Isopropyl sind, bevorzugt sind R₅ und R₆ ein H.

Weitere bevorzugte Verbindungen der allgemeinen Formel (I) sind dadurch gekennzeichnet, dass P1 ein 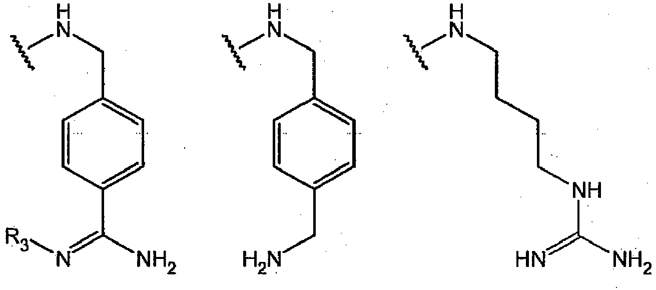 ist, wobei R₃ definiert ist wie zuvor, aber bevorzugt ein H ist.

Weitere bevorzugte Verbindungen der allgemeinen Formel (I) sind dadurch gekennzeichnet, dass m gleich 0 oder 1 ist.

Besonders wirksame Inhibitoren gemäß der allgemeinen Formel (I) besitzen folgende Strukturen: 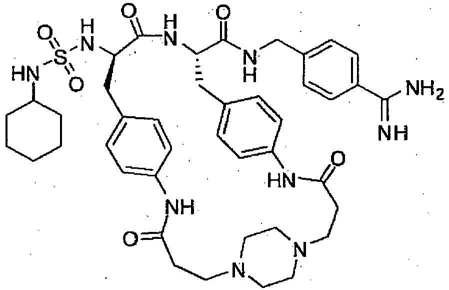 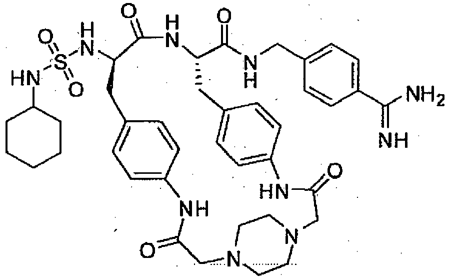 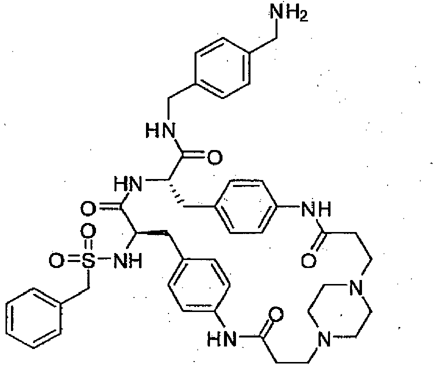 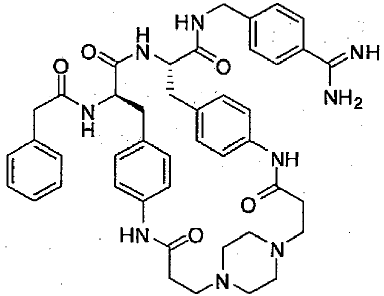 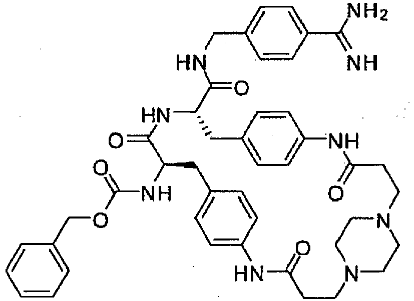 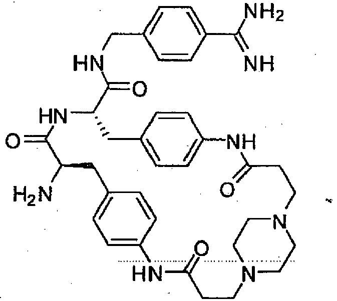 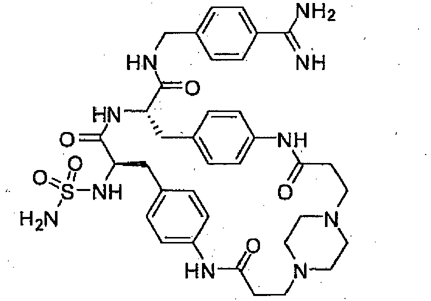 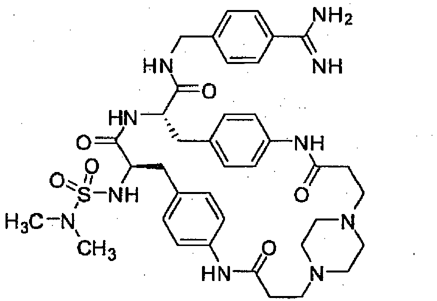 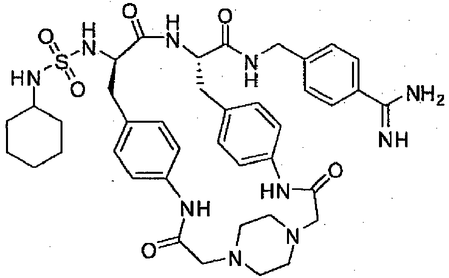 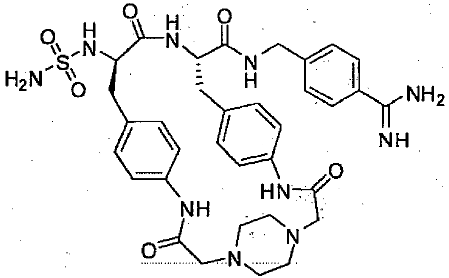 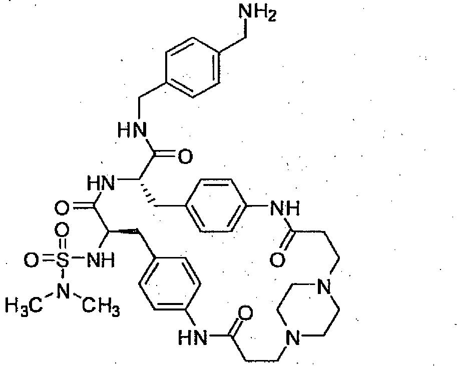 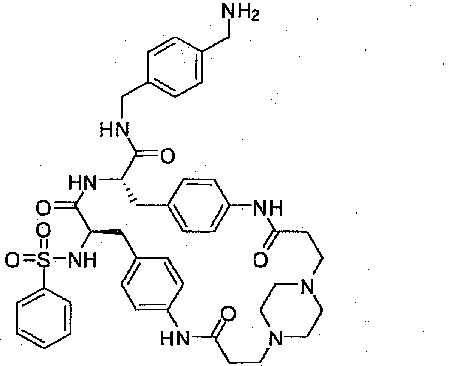 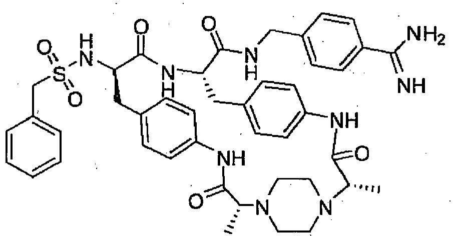 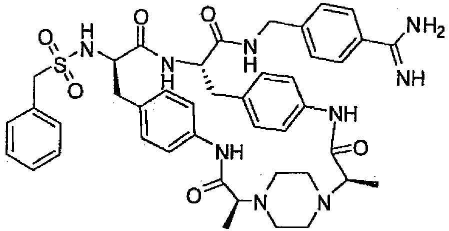 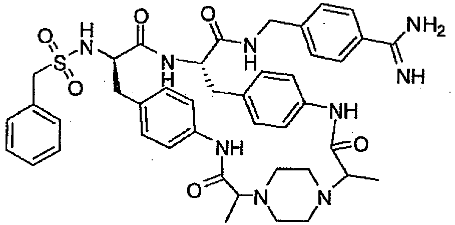 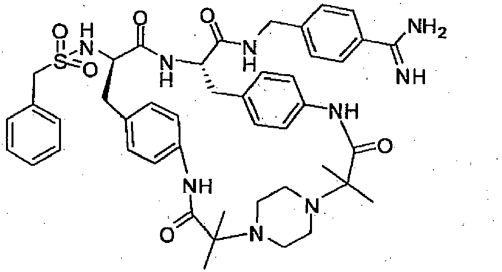 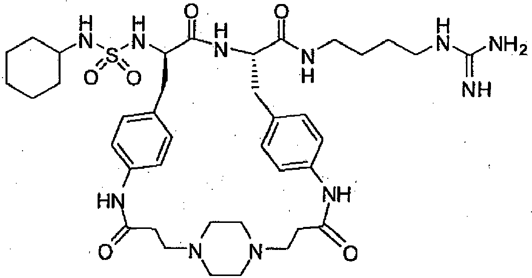 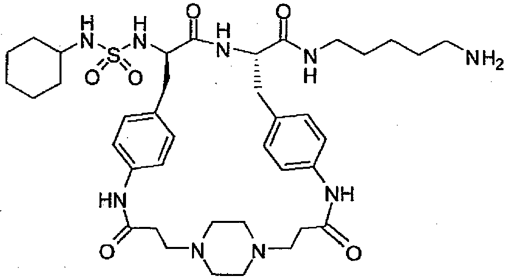 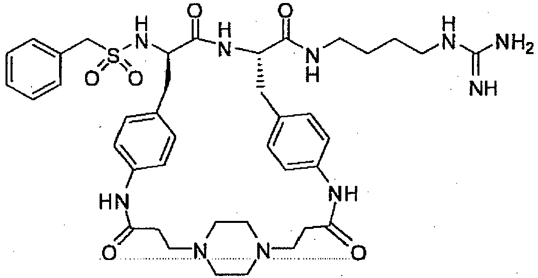 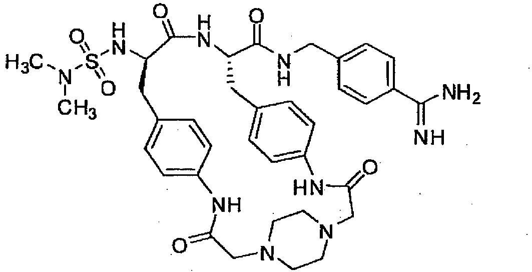 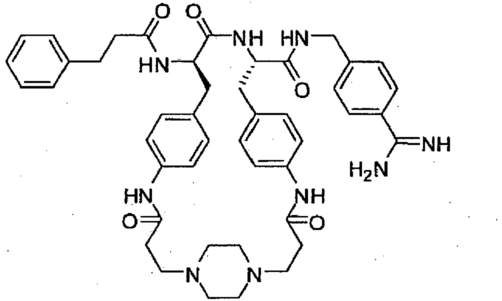 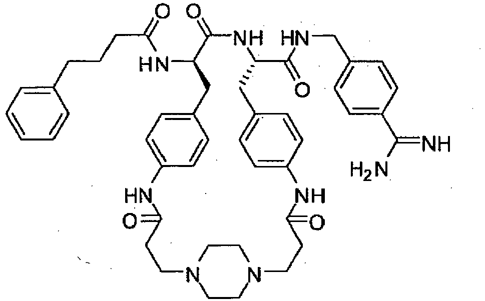 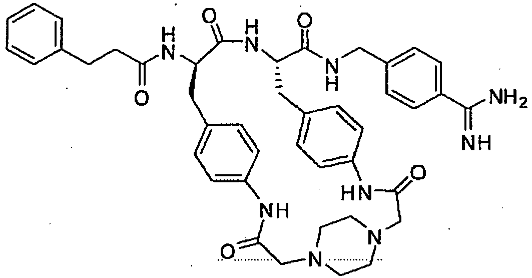 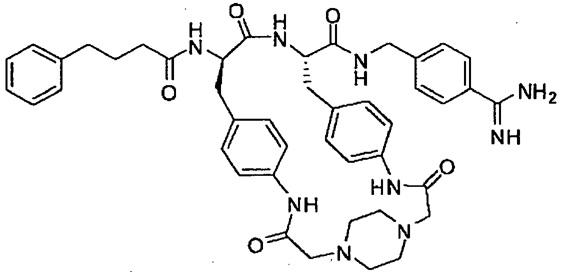 wobei die basischen und gegebenenfalls vorhandenen sauren Gruppen in einer physiologisch verträglichen Salzform vorliegen.

Die Hemmstoffe gemäß der allgemeinen Formel (I) können als Arzneimittel verwendet werden, entweder in Form einer Tablette, eines Dragees, einer Kapsel, eines Pellets, eines Suppositoriums, einer Lösung, insbesondere einer Injektions- oder Infusionslösung, von Augen-, Nasen- oder Ohrentropfen, eines Saftes, einer Emulsion oder Suspension, eines Globuli, eines Styli, eines Aerosols, eines Aerosol-Sprays, eines Puders, einer Paste, einer Creme oder einer Salbe, ganz besonders bevorzugt sind allerdings Infusions- und Injektionslösungen.

Diese Arzneimittel können verwendet werden, um den Blutverlust bei hyperfibrinolytischen Zuständen zu reduzieren. Sie sind besonders geeignet, um den Blutverlust bei chirurgischen Eingriffen, besonders bei Herzoperationen und Organtransplantationen zu vermindern. Dadurch kann der Verbrauch von Blutkonserven reduziert werden.

Die Verbindungen gemäß der allgemeinen Formel (I) können auch als Zusatzstoff für die Herstellung von Fibrinklebern eingesetzt werden.

Die Verbindungen gemäß der allgemeinen Formel (I) können auch als Arzneimittel zur Behandlung von Krebs oder der Hemmung der Metastasierung eingesetzt werden.

Eine weitere Anwendung dieser Plasmininhibitoren ist die Behandlung der proliferativen Vitreoretinopathie (PVR), oder Ihre Verwendung als Antidot bei der Thrombolysetherapie mittels Plasmin oder Plasminogenaktivatoren, falls zu starke Blutungen auftreten.

Die Verbindungen gemäß der allgemeinen Formel (I) können nicht nur als Arzneimittel zur Behandlung von Menschen, sondern auch bei Tieren eingesetzt werden, z.B., zur Reduktion von Blutverlusten bei chirurgischen Eingriffen, besonders bei Herzoperationen und Organtransplantationen, für die Herstellung von Fibrinklebern oder die Behandlung von Tumoren oder der Hemmung der Metastasierung.

Die Verbindungen gemäß der allgemeinen Formel (I) können auch für die Hemmung der Serinproteasen Plasmin für in vitro Anwendungen eingesetzt werden, beispielsweise für diagnostische Zwecke oder biochemische Untersuchungen.

Gegenstand der Erfindung sind auch pharmazeutisch geeignete bzw. verträgliche Salze der Verbindungen gemäß Formel (I). Diese Salze müssen ein pharmazeutisch verträgliches Anion oder Kation aufweisen. Geeignete pharmazeutisch verträgliche Säureadditionssalze der erfindungsgemäßen Verbindungen sind z. B. Salze anorganischer Säuren, wie Salzsäure, Bromwasserstoff-, Phosphor-, Methaphosphor-, Salpeter-, Sulfon- und Schwefelsäure oder organischer Säuren, wie z. B. Essigsäure, Benzolsulfon-, Benzoe-, Zitronen-, Ethansulfon-, Fumar-, Glucon-, Glykol-, Isethion-, Milch-, Lactobion-, Malein-, Methansulfon-, Bernstein-, p-Toluonsulfon-, Wein- und Trifluoressigsäure. Für medizinische Zwecke wird in besonders bevorzugter Weise das Chlor- oder Acetatsalz verwendet. Geeignete phamazeutisch verträgliche basische Salze sind z. B. Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der im folgenden verwendete Begriff "physiologisch funktionelles Derivat" bezeichnet jedes physiologisch verträgliche Derivat, z. B. einen Ester, der bei Verabreichung an einen Säuger, wie z. B. den Menschen, in der Lage ist, (direkt oder indirekt) eine Verbindung der Formel I oder einen aktiven Metaboliten hiervon zu bilden. Zu den physiologisch funktionellen Derivaten zählen auch Prodrugs. Solche Prodrugs können *in vivo* metabolisiert werden. Diese Prodrugs können selber wirksam sein oder nicht. Geeignete Prodrugs für Benzamidin-Gruppen sind beispielsweise Verbindungen, bei denen das Amidin als Hydroxyamidin, Methoxyamidin, Acetylhydroxyamidin, Alkyloxycarbonylamidin, speziell als Hexyloxycarbonylamidin vorliegt.

Die erfindungsgemäßen Verbindungen können auch in verschiedenen polymorphen Formen vorliegen, z. B. als amorphe und kristalline polymorphe Formen und sind ein weiterer Aspekt der Erfindung.

Nachfolgend beziehen sich alle Verweise auf Verbindungen gemäß Formel (I) wie vorstehend beschrieben, sowie Salze und Solvate.

Die vorliegende Erfindung betrifft auch die Verbindungen der Formel (I) zür Verwendung als Hemmstoffe des Plasmins. Auch eine pharmazeutische Zusammensetzung enthaltend eine Verbindung der Formel (I) ist Gegenstand dieser Erfindung. Die Menge der Verbindung gemäß Formel (I), die erforderlich ist, um den gewünschten biologischen Effekt zu erreichen, ist abhängig von einer Reihe von Faktoren, z. B. der gewählten spezifischen Verbindung, der beabsichtigten Verwendung, der Art der Verabreichung und dem klinischen Zustand des Patienten.

Im Allgemeinen liegt die Tagesdosis im Bereich von 0,03 mg bis 1000 mg (typischerweise von 3 mg bis 100 mg) pro Tag pro Kilogramm Körpergewicht, z. B. 3-10 mg/kg/Tag. Eine intravenöse Dosis kann z. B. im Bereich von 0,03 mg bis 3,0 mg/kg liegen, die geeigneterweise als Infusion von 10 ng bis 100 µg pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z. B. von 1 mg bis 3 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht der dem Salz zugrunde liegenden freien Verbindung. Zur Prophylaxe oder Therapie der oben genannten Zustände können die Verbindungen gemäß Formel (I) selbst als Verbindung verwendet werden, vorzugsweise liegen sie jedoch mit einem verträglichen Träger oder Hilfsstoff in Form einer pharmazeutischen Zusammensetzung vor. Der Träger bzw. Hilfsstoff muss natürlich verträglich sein, in dem Sinn, dass er mit den anderen Bestandteilen der Zusammensetzung kompatibel ist und nicht gesundheitsschädlich für den Patienten ist.

Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im Wesentlichen darin bestehen, dass die Bestandteile mit pharmakologisch verträglichen Träger- und/oder Hilfsstoffen gemischt werden.

Erfindungsgemäße pharmazeutische Zusammensetzungen sind insbesondere solche, die für orale, rektale, topische, perorale (z. B. sublinguale) und parentale (z. B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch Aerosole sind geeignete pharmazeutische Zusammensetzungen für die erfindungsgemäßen Verbindungen, die auch in Form von Aerosol-Sprays besonders für die Behandlung und Prophylaxe von Erkrankungen des Respirationstraktes geeignet sind. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete Pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im Allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird.

So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Füllstoff und/oder einem (mehreren) oberflächenaktiven/dispergierenden Mittel in einer geeigneten Maschine hergestellt werden. Geformte Tabletten können durch Formen der pulverförmigen, mit einem inerten flüssigen Füllmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose oder Gummi arabicum oder Tragant, und Pastillen, die die Verabreichung einer inerten Basis wie Gelatine und Glycerin oder Saccharose und Gummi arabicum umfassen.

Geeignete pharmazeutische Zusammensetzungen für die parentale Verabreichung umfassen vorzugsweise sterile wässrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich eine Verabreichung auch subkutan, intramuskulär oder intradermal als Injektion erfolgen kann.

Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im Allgemeinen von 0,1 bis 5 Gew.-% der aktiven Verbindung.
Bezüglich der weiteren Formulierung wird auf gängige Handbücher verwiesen.

Die Erfindung betrifft auch Verfahren zur Herstellung von pharmazeutischen Zusammensetzungen, bei denen man eine oder mehrere Verbindungen der allgemeinen Formel (I) mit geeigneten Träger- und Hilfsstoffen (siehe oben) vermischt.

Die Erfindung wird durch die folgenden Ausführungsbeispiele näher erläutert.

### Ausführungsbeispiele

### Methoden zur Analyse der Verbindungen

### Analytische HPLC

Zur analytischen reversed-phase-HPLC wurde eine HPLC-Anlage LC-10A der Firma Shimadzu, bestehend aus den Teilsystemen CTO-10A Säulenofen, LC-10ATvp Pumpen (2 x), DGU-14A Degaser, SIL-10Axl Autoinjektor, SCL-10Avp Systemcontroller, SPD-M10Avp Photodiodenarraydetektor und einer Säule 250/4,6 Nucleodur 100-5 C18 ec der Firma Macherey-Nagel (Düren, Deutschland), unter Benutzung der zugehörigen Software Shimadzu CLASS-VP, Version 7.2.1, verwendet. Die Detektion erfolgte bei 220 nm. Als Elutionsmittel dienten Wasser mit 0,1 % TFA (Laufmittel A) und Acetonitril mit 0,1 % TFA (Laufmittel B) bei einer Flussrate von 1 mL/min und einem linearen Gradienten (Anstieg von 1 % B/min). Die jeweiligen Startbedingungen (% B) sind bei den Synthesen angegeben.

### Präparative HPLC

Zur präparativen RP-HPLC wurde eine HPLC-Anlage der Firma Varian, bestehend aus den Teilsystemen Varian PrepStar Model 218 präparative Pumpen (2 x), Varian ProStar Model 320 UV-Vis Detektor, Varian Fraktionssammler Model 701, und einer Säule VP 250/32 Nucleodur 100-5 C8 ec der Firma Macherey-Nagel (Düren, Deutschland), unter Benutzung der zugehörigen Star-Software V. 6.0 verwendet. Einige Trennungen erfolgten an einer Prontosil-C18-Säule (250/32, 120-5-C18-SH, Bischoff Chromatography). Die Detektion erfolgte bei 220 nm. Als Elutionsmittel dienten ebenfalls Wasser mit 0,1 % TFA (Laufmittel A) und Acetonitril mit 0,1 % TFA (Laufmittel B) bei einer Flussrate von 20 mL/min und einem geeigneten Gradienten (Anstieg 1 % B in 2 min).

### Massenspektroskopie

Die Spektren wurden mit einem Gerät der Firma Applied Biosystems (QTrap 2000) oder einem Spektrometer vom Typ Autospec der Firma Micromass aufgenommen.

### Verwendete Abkürzungen

| | |
|---|---|
| 4-AMBA | 4-Amidinobenzylamid |
| Boc | tert.-Butyloxycarbonyl |
| Chas | Cyclohexylaminosulfonyl |
| CKIBE | Chlorkohlensäureisobutylester |
| DIPEA | Diisopropylethylamin |
| DCM | Dichlormethan |
| DMF | N,N-Dimethylformamid |
| EtAc | Essigsäureethylester |
| HATU | 2-(1H-7-Azabenzotriazol-1-yl)-1,1,3,3-tetramethyl uronium hexafluorophosphate Methanaminium |
| HPLC | Hochleistungs-Flüssigkeitschromatographie |
| i.V. | im Vakuum |
| LM | Lösungsmittel |
| Me | Methyl |
| MeCN | Acetonitril |
| MS | Massenspektroskopie |
| NMM | N-Methylmorpholin |
| Phe(4-NO₂) | 4-Nitrophenylalanin |
| RT | Raumtemperatur |
| TFA | Trifluoressigsäure |
| TMS-Cl | Trimethylsilylchlorid |

Die verwendeten Chemikalien, Lösungsmittel, Reagentien und Aminosäurederivate wurden von den Firmen Aldrich, Fluka, Acros, Bachem, Iris Biotech, Peptech und Novabiochem bezogen.

### Beispiel 1: Synthese des Inhibitors 1

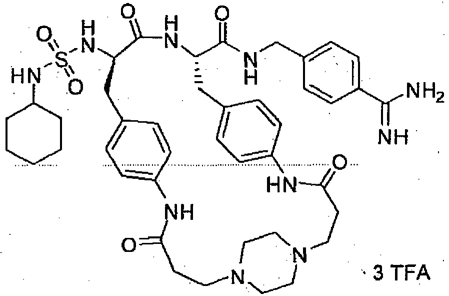

### 1a) Chas-D-Phe(4-NO₂)-OH

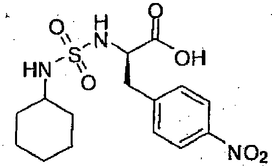

2,0 g (9,52 mmol) H-D-Phe(4-NO₂)-OH wurde in 25 ml trockenem DCM suspendiert und mit 2,68 ml (20,93 mmol) TMS-Cl und 3,64 ml (20,93 mmol) DIPEA versetzt. Der Ansatz wurde eine Stunde unter Rückfluß gekocht, anschließend wurden bei 0 °C portionsweise 2,54 g (12,85 mmol) Cyclohexylsulfamoylchlorid (G. Weiß, G. Schulze, Liebigs Ann. Chem., 1969, 729, 40-51) innerhalb von 35 min zugegeben, dabei wurde der pH-Wert auf 7-8 durch Zugabe von insgesamt 2,23 ml (12,85 mmol) DIPEA eingestellt. Die Lösung wurde für 4 h auf dem Eisbad und über Nacht bei RT gerührt. Das LM wurde i.V. entfernt und der braune, zähe Rückstand in 5% KHSO₄-Lsg./EtAc aufgenommen. Die wässrige Phase wurde 2 x mit Ethylacetat extrahiert. Die vereinten org. Phasen wurden 2 x mit 5 % KHSO₄- und 3 x mit gesättigter NaCl-Lösung gewaschen. Die org. Phase wurde mit MgSO₄ getrocknet, filtriert und das LM i.V. entfernt.
Ausbeute: 3,095 g hellbrauner, amorpher Feststoff (HPLC: 38,0 min, Start bei 10 % B; MS: ber.: 371,12 gef.: 372,17 [M+H]⁺).

### 1 b) Chas-D-Phe(4-NO₂)-Phe(4-NO₂)-OMe

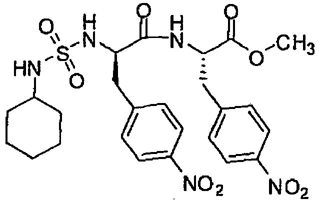

2,0 g (5,39 mmol) Chas-D-Phe(4-NO₂)-OH und 1,42 g (5,39 mmol) H-Phe(4-NO₂)-OMe·HCl wurden in 30 ml DMF suspendiert und bei 0 °C mit 2,82 g (5,39 mmol) PyBOP und 2,81 ml (16,16 mmol) DIPEA versetzt. Nach 2 h rühren bei 0 °C wurde das LM i.V. entfernt und der braune Rückstand in 5%-KHSO₄-Lsg./EtAc aufgenommen. Die org. Phase wurde 3 x mit 5%-KHSO₄-Lsg., 1 x mit ges. NaCl-Lsg., 3 x mit ges. NaHCO₃-Lsg. und 3 x mit ges. NaCl-Lsg. gewaschen, mit MgSO₄ getrocknet, filtriert und das LM i.V. entfernt.
Ausbeute: 3,66 g braunes, zähes Öl (HPLC: 50,0 min, Start bei 10 % B; MS: ber.: 577,18 gef.: 600,29 [M+Na]⁺).

### 1c) Chas-D-Phe(4-NH₂)-Phe(4-NH₂)-OMe

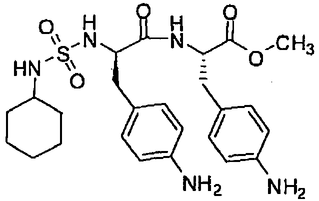

1,0 g (1,47 mmol) Chas-D-Phe(4-NO₂)-Phe(4-NO₂)-OMe wurde in 200 ml 90% Essigsäure gelöst, mit 101 mg Pd-C versetzt, 3 x sekuriert und über Nacht mit Wasserstoff hydriert. Der Katalysator wurde abfiltriert und das LM i.V. entfernt. Das rote, zähe Öl wurde in 8 ml 25 % Laufmittel B gelöst, durch einen 0,2 µm Filter filtriert und mittels präp. HPLC gereinigt (Start bei 10% B). Die Produkt enthaltenen Fraktionen wurden vereint, das LM i.V. entfernt, der Rückstand in 40 % t-Butanol/Wasser gelöst und lyophilisiert.
Ausbeute: 328 mg hellgelbe, lyoph. Substanz (HPLC: 18,4 min, Start bei 10 % B; MS: ber.: 517,24 gef.: 518,13 [M+H]⁺).

### 1d) N,N'-1,4-Piperazindipropansäure

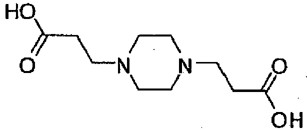

5.g (25,7 mmo) Piperazin 6 H₂O wurde in 140 ml 10% NaOH-Lösung gelöst und mit 8,08 g (52,8 mmol) 3-Brompropansäure versetzt. Die gelbe, klare Lösung wurde über Nacht bei RT gerührt. Die Lösung wurde mit 37% HCl angesäuert, dabei fiel das Produkt als hellgelbe kristalline Substanz. aus. Der Ansatz wurde über Nacht bei 4 °C gelagert, das Präzipitat abfiltriert, mit Wasser gewaschen und i.V. getrocknet.
Ausbeute: 4,58 g hellgelb, krist. Substanz (MS: berechnet: 230,13, gef. 229,17 [M-H]⁻)

### 1e)

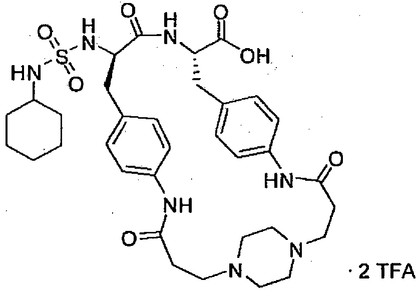

100 mg (0,193 mmol) Chas-D-Phe(4-NH₂)-Phe(4-NH₂)-OMe (1c) und 44,5 mg (0,193 mmol) N,N'-1,4-Piperazindipropansäure wurden in 50 ml DMF suspendiert und auf dem Eisbad gerührt. Nach Zugabe von 202 mg (0,386 mmol) PyBOP und 134,4 µl (0,773 mmol) DIPEA wurde der Ansatz bei RT über Nacht gerührt und anschließend das LM i.V. entfernt. Das gelbe Öl wurde mit je 2 ml Ethanol und 1 NaOH versetzt, der Ansatz für 2,5 h bei RT gerührt und anschließend mit TFA neutralisiert. Das LM wurde i.V. entfernt, der weiße Rückstand in 7 ml 30% Laufmittel B gelöst und mittels präparativer HPLC gereinigt (Start bei 15% B). Die Produkt enthaltenen Fraktionen wurden vereint, das LM i.V. entfernt, der Rückstand in 40% tert.-Butanol/Wasser gelöst und lyophilisiert.
Ausbeute: 45,3 mg weiße, lyoph. Substanz (HPLC: 23,9 min, Start bei 10 % B, MS: ber.: 669,29 gef.: 670,24 [M+H]⁺).

### 1)

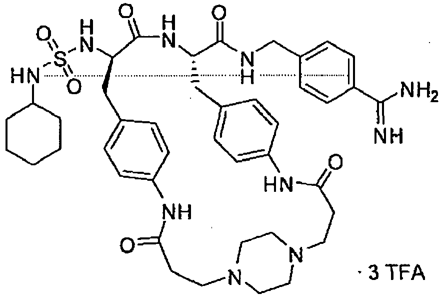

35 mg (0,0378 mmol) des Produktes 1e wurden in 3 ml DMF gelöst und auf -15 °C abgekühlt. Nach Zugabe von 4,91 µl (0,0378 mmol) CKIBE und 12,5 µl (0,113 mmol) NMM wurde der Ansatz für 15 min gerührt und danach 12,8 mg (0,0567 mmol) 4-AMBA·2 HCl und 4,16 µl NMM zugegeben. Der Ansatz wurde eine Stunde bei -15 °C und über Nacht bei RT gerührt und anschließend das LM i.V. entfernt. Der gelbe Rückstand wurde in 8 ml 30% Laufmittel B gelöst und mittels präparativer HPLC gereinigt (Start bei 15% B). Die Produkt enthaltenen Fraktionen wurden vereint, das LM i.V. entfernt, der Rückstand in 40 % t-Butanol/Wasser gelöst und lyophilisiert.
Ausbeute: 40,5 mg weiße, lyoph. Substanz (HPLC: 21,7 min, Start bei 10 % B, MS: ber.: 828,41 gef.: 829,59 [M+H]⁺, 415,47 [M+2H]⁺⁺).

### Beispiel 2: Synthese des Inhibitors 2

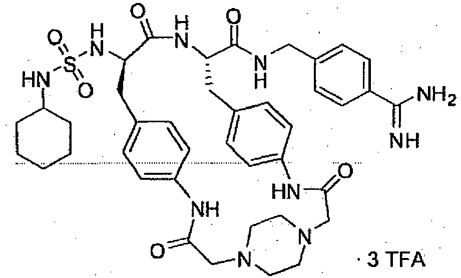

Die Synthese des Inhibitors 2 erfolgte analog der Strategie zur Synthese des Inhibitors 1, nur das im Schritt 1e für die Zyklisierungsreaktion N,N'-Piperazindiessigsäure (Li Shen et al. ChemEurJ, 2006, 12, 4393-4396) eingesetzt wurde.

Ausbeute: 29 mg weiße, lyoph. Substanz (HPLC: 21,1 min, Start bei 10 % B, MS: ber.: 800,38 gef.: 801,38 [M+H]⁺).

### Beispiel 3: Synthese des Inhibitors 3

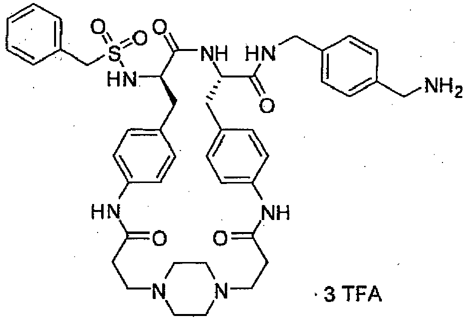

### 3a) Bzls-D-Phe(4-NO₂)-OH

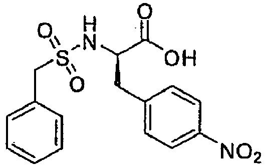

5,0 (23,8 mmol H-D-Phe(4-NO₂)-OH (Peptech) wurde in 50 ml trockenem DCM suspendiert und mit 6,5 ml (52,4 mmol) TMS-Cl und 9,1 ml (52,4 mmol) DIPEA versetzt. Der Ansatz wurde für 1 h unter Rückfluß gekocht und dann auf 0°C abgekühlt. Anschließend wurden 5,02 g (26,3 mmol Benzylsulfonylchlorid in mehreren Portionen innerhalb von 1 h zugegeben, und der pH-Wert auf 8-9 durch Zugabe von 4,6 ml (26,4 mmol) DIPEA eingestellt. Der Ansatz wurde noch 1 h bei 0°C und über Nacht bei RT gerührt. Das LM wurde i.V. entfernt und der braune Rückstand in 5% KHSO₄-Lsg/EtAc aufgenommen. Die wässrige Phase wurde 2 x mit EtAc extrahiert und die vereinten organischen Phasen 3 x mit 5% KHSO₄-Lsg. und 3 x mit ges. NaCl-Lsg. gewaschen, mit MgSO4 getrocknet, filtriert und das LM i.V. entfernt.

Das braune Öl wurde in 250 ml EtAc gelöst und mit 7,1 ml (35,5 mmol) DCHA versetzt. Sie Suspension wurde mehrere Tage bei 4 °C aufbewahrt. Die braunen Kristalle wurden abfiltriert, mit EtAc und Diethylether gewaschen und im Vakuum getrocknet.
Ausbeute: 8,2 g hellbraune, krist. Substanz als DCHA-Salz (HPLC: 38,7 min, Start bei 10 % B)

2,7 g vom DCHA-Salz wurde in 5% KHSO₄-Lsg/EtAc aufgenommen und die saure wässrige Phase 3 x mit EtAc extrahiert. Die vereinten organischen Phasen wurden 3 x mit ges. NaCl-Lsg. gewaschen, mit MgSO₄ getrocknet, filtriert und das LM i.V. entfernt.
Ausbeute: 1,8 g hellbraunes Öl (HPLC: 38,7 min, Start bei 10 % B, MS: ber.: 364,07 gef.: 363,1 [M+H]⁻).

### 3b) Bzls-D-Phe(4-NO₂)-Phe(4-NO₂)-OMe

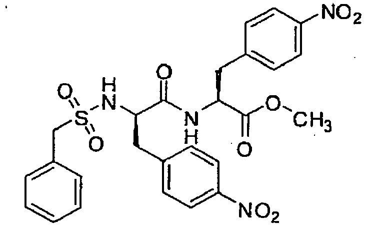

1,8 g (4,94 mmol) Bzls-D-Phe(4-NO₂)-OH (3a) und 1,29 g (4,94 mmol) H-Phe(4-NO₂)-OMe·HCl (Aldrich) wurden in 30 ml DMF suspendiert und auf dem Eisbad gerührt. Nach Zugabe von 2,57 g (4,94 mmol) PyBOP und 1,72 ml (9,88 mmol) DIPEA (pH 7-8) wurde der Ansatz 15 min bei 0 °C und 3 h bei RT gerührt. Das LM wurde i.V. entfernt und das dunkelgelbe Öl in 5% KHSO₄-Lsg./EtAc aufgenommen. Die organische Phase wurde 3 x mit 5% KHSO₄-Lsg., 1 x mit ges. NaCl-Lsg., 3 x mit ges. NaHCO₃-Lsg. und 3 x mit ges. NaCl-Lsg. gewaschen. Die org. Phase wurde mit MgSO₄ getrocknet, filtriert und das LM i.V. entfernt.
Ausbeute: 3,55 g brauner, amorpher Rückstand mit Verunreinigungen (HPLC: 48,4 min, Start bei 10 % B, MS: ber.: 570,57 gef.: 571,23 [M+H]⁺).

### 3c) Bzls-D-Phe(4-NH₂)-Phe(4-NH₂)-OMe

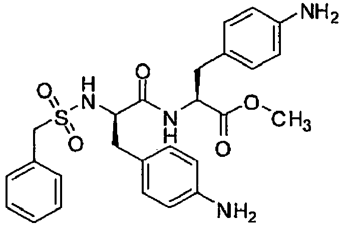

2,82 g Bzls-D-Phe(4-NO₂)-Phe(4-NO₂)-OMe (3b) wurde in 500 ml 90% Essigsäure gelöst, Zinkstaub zugegeben, 4 h bei RT gerührt und anschließend das LM i.V. entfernt. Der gelbe Rückstand wurde in MeCN/Wasser (9/1, v/v) suspendiert, die ungelösten Salze durch Zentrifugation und das LM i.V. entfernt. Der Rückstand wurde mittels präp. HPLC gereinigt (Start bei 5% B). Die Produkt enthaltenen Fraktionen wurden vereint, die Lösung eingeengt und lyophilisiert. Ausbeute: 2.02 g hellgelbe, lyoph. Substanz (HPLC: 25,0 min, Start bei 1 % B, MS: ber.: 510,61 gef.: 511,27 [M+H]⁺).

### 3d)

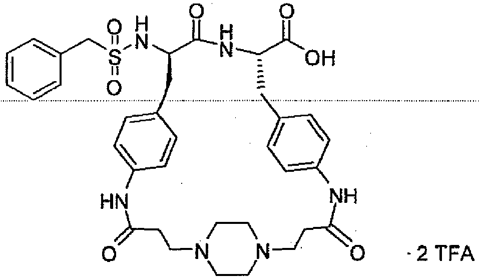

500 mg (0,979 mmol) Bzls-D-Phe(4-NH₂)-Phe(4-NH₂)-OMe (3c) und 225 mg (0,979 mmol) N,N'-1,4-Piperazindipropansäure wurden in 250 ml DMF suspendiert und auf dem Eisbad gerührt. Nach Zugabe von 1,02 g (1,96 mmol) PyBOP und 682 µl (3,92 mmol) DIPEA wurde der Ansatz über Nacht gerührt und anschließend das LM i.V. entfernt. Das dunkelrote Öl wurde mit je 5 ml Ethanol und 1 N NaOH gelöst. Der Ansatz wurde für 4 h bei RT gerührt und anschließend mit TFA neutralisiert. Das LM wurde i.V. entfernt, der weiße Rückstand in 2 x 8 ml 25% B gelöst und mittels präparativer HPLC gereinigt (Start bei 15% B). Die Produkt enthaltenen Fraktionen wurden vereint, das LM i.V. entfernt, der Rückstand in 40% t-Butanol/Wasser gelöst und lyophilisiert.
Ausbeute: 204 mg weiße, lyoph. Feststoff (HPLC: 22,5 min, Start bei 10 % B, MS: ber.: 690,28 gef.: 691,33 [M+H]⁺, 713,36 [M+Na]⁺).

### 3)

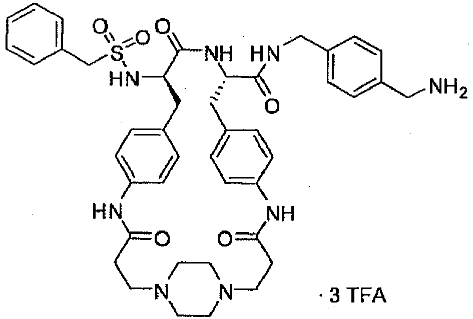

100 mg (0,109 mmol) der Verbindung 3d wurde in 4 ml DMF gelöst und auf -15 °C abgekühlt. Nach Zugabe von 36 µl (0,327 mmol) NMM und 15 µl (0,109 mmol) CKIBE wurde die Lösung für 15 min gerührt und dann 40 mg (0,169 mmol) Boc-p-diaminoxylol und 12 µl (0,109 mmol) NMM zugegeben. Der Ansatz wurde eine Stunde bei 0°C und über Nacht bei RT gerührt. Das LM wurde i.V, entfernt, der Rückstand mit 1 ml 90% TFA versetzt und für 2 h bei RT gerührt. Nach entfernen des LM i.V. wurde der Rückstand in 8 ml 30% B gelöst und mittels präp. HPLC gereinigt (Start bei 10% B). Die Produkt enthaltenen Fraktionen wurden vereint, das LM i.V. entfernt, der Rückstand in 80% t-Butanol/Wasser gelöst und lyophilisiert.
Ausbeute: 39 mg weißer, lyoph. Feststoff (HPLC: 20,1 min, Start bei 10 % B, MS: ber.: 808,37 gef.: 809,10 [M+H]⁺, 405,34 [M+2H]⁺⁺/2).

### Beispiel 4: Synthese des Inhibitors 4

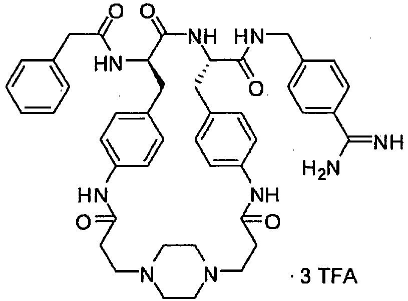

### 4a) Boc-D-Phe(4-NO₂)-Phe(4-NO₂)-OMe

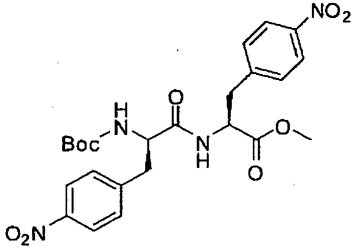

1,0 g (3,22 mmol) Boc-D-Phe(4-NO₂)-O (Iris Biotech) und 0,84 g (3,22 mmol) H-Phe(4-NO₂)-OMe·HCl (Aldrich) wurden in 25 ml DMF suspendiert und auf dem Eisbad gerührt. Nach Zugabe von 1,66 g (3,22 mmol) PyBOP und 1,67 ml (9,66 mmol) DIPEA (pH 7-8) wurde der Ansatz 2,5 h bei 0 °C gerührt. Das LM wurde i.V. entfernt und das gelbe Öl in 5% KHSO₄-Lsg./EtAc aufgenommen. Die organische Phase wurde 3 x mit 5% KHSO4-Lsg., 1 x mit ges. NaCl-Lsg., 3 x mit ges. NaHCO₃-Lsg. und 3 x mit ges. NaCl-Lsg. gewaschen. Die org. Phase wurde mit MgSO₄ getrocknet, filtriert und das LM i.V. entfernt.
Ausbeute: 1,76 g gelber, amorpher Rückstand mit Verunreinigungen (HPLC: 52,5 min, Start bei 10% B, MS: ber.: 516,19 gef.: 539,20 [M+Na]⁺).

### 4b) Boc-D-Phe(4-NH₂)-Phe(4-NH₂)-OMe

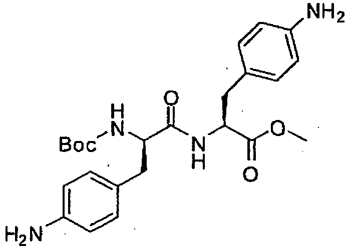

1,6 g (3,00 mmol) Boc-D-Phe(4-NO₂)-Phe(4-NO₂)-OMe (4a) wurde in 150 ml 90 % Essigsäuregelöst, 160 mg Pd-C zugegeben, 3 x sekuriert und über Nacht bei RT hydriert. Nach filtrieren der Suspension wurde das LM i.V. entfernt. Der Rückstand wurde in ges. NaHCO₃-Lsg./EtAc aufgenommen und die org. Phase 3 x mit ges. NaHCO₃-Lsg. und 2 x mit ges. NaCl-Lsg. gewaschen. Nach dem Trocknen der org. Phase mit MgSO₄ wurde filtriert und das LM i.V. entfernt.
Ausbeute: 1,36 g hellbrauner, amorpher Feststoff (HPLC: 15,6 min, Start bei 10% B; MS: ber.: 456,24 gef.: 479,21 [M+Na]⁺).

### 4c)

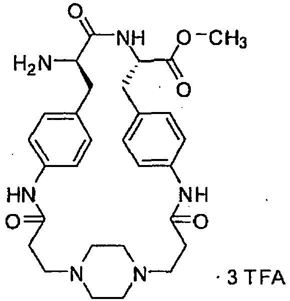

1,26 g (2,76 mmol) Boc-D-Phe(4-NH₂)-Phe(4-NH₂)-OMe (4b) und 630 mg (2,760 mmol) N,N'-1,4-Piperazindipropansäure werden in 630 ml DMF suspendiert. Nach 5 min kühlen bei 0 °C wurden 2,87 g (5,52 mmol) PyBOP und 1,92 ml (11,04 mmol) DIPEA hinzugefügt (pH 7-8) und der Ansatz für 6 h gerührt. Nach HPLC-Kontrolle (unvollständiger Umsatz) wurden weitere 1,15 g (2,210 mmol) PyBOP und 768 µl (4,414 mmol) DIPEA bei 0 °C hinzugefügt und der Ansatz 1 h bei 0°C und über Nacht bei RT gerührt. Das LM wurde i.V. entfernt, das gelbe Öl mit 15 ml TFA versetzt und für 1 h, unter gelegentlichem Schwenken, stehen gelassen. Das Produkt wurde als TFA-Salz mit Diethylether gefällt, das LM entfernt und das Produkt i.V. getrocknet. Der rötliche Rückstand wurde in 20% Laufmittel B gelöst und mittels präp. HPLC gereinigt (Start bei 0% B). Die Produkt enthaltenen Fraktionen wurden vereint, das LM i.V. eingeengt und lyophilisiert.
Ausbeute: 360 mg weißer, lyoph. Feststoff (HPLC: 11,3 min, Start bei 10% B; MS: ber.: 550,29 gef.: 551,38 [M+H]⁺, 573,35 [M+Na]⁺).

### 4d)

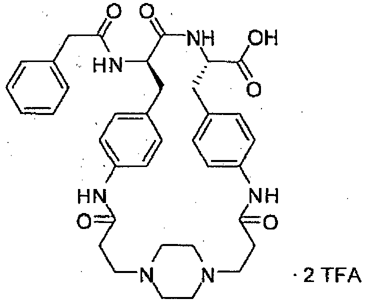

15,3 mg (0,112 mmol) Phenylessigsäure wurde in 3 ml DMF gelöst und auf -15 °C abgekühlt. Nach Zugabe von 12,3 µl (0,112 mmol) NMM und 14,6 µl (0,112 mmol) CKIBE wurde die Lösung für 15 min gerührt und dann 100 mg (0,112 mmol) der Verbindung 4c und 36,9 µl (0,336 mmol) NMM zugegeben. Der Ansatz wurde 1 h bei -15°C und über Nacht bei RT gerührt. Das LM wurde i.V, entfernt, der Rückstand mit je 2 ml EtOH und 1 N NaOH versetzt und für 3 h bei RT gerührt. Nach dem Neutralisieren mit TFA wurde das LM i.V. entfernt, der Rückstand in 8 ml 35% Laufmittel B gelöst und mittels präp. HPLC gereinigt (Start bei 15% B). Die Produkt enthaltenen Fraktionen wurden vereint, das LM i.V. entfernt, der Rückstand in 40% tert.-Butanol/Wasser gelöst und lyophilisiert.
Ausbeute: 45 mg weiße, lyoph. Substanz (HPLC: 21,4 min, Start bei 10 % B, MS: ber.: 654,32 gef.: 655,31 [M+H]⁺, 677,09 [M+Na]⁺).

### 4)

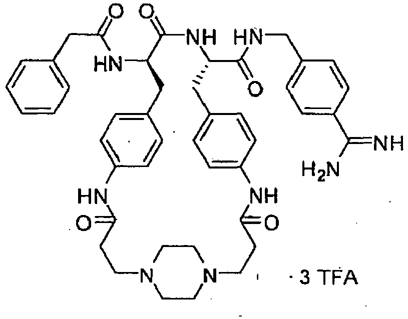

40,1 mg (0,0453 mmol) der Verbindung 4d wurden in 2 ml DMF gelöst und auf -15 °C gekühlt. Nach Zugabe von 15,0 µl (0,136 mmol) NMM und 5,9 µl (0,0453 mol) CKIBE wurde die Lösung für 15 min gerührt und anschließend 15,1 mg (0,0680 mmol) 4-AMBA·2 HCl und 5 µl (0,0453 mmol) NMM zugegeben. Der Ansatz wurde 1 h bei -15°C und über Nacht bei RT gerührt. Das wurde LM i.V. entfernt, der Rückstand in 8 ml 30% Laufmittel B gelöst und mittels präp. HPLC gereingt (Start bei 10% B). Die Produkt enthaltenen Fraktionen wurden vereint, das LM i.V. entfernt, der Rückstand in 40% t-Butanol/Wasser gelöst und lyophilisiert.
Ausbeute: 16,5 mg weißer, lyoph. Feststoff (HPLC: 19,3 min, Start bei 10 % B, MS: ber.: 785,40 gef.: 786,30 [M+H]⁺, 393,81 [M+2H]⁺⁺).

### Beispiel 5: Synthese des Inhibitors 5

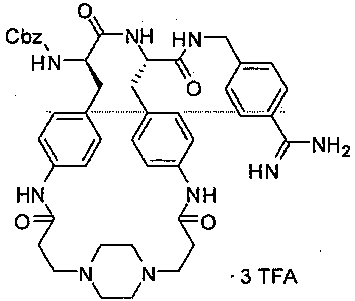

### 5a)

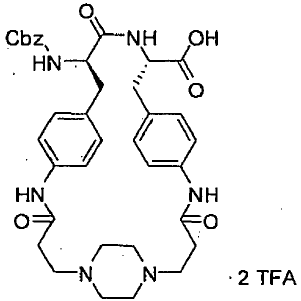

150 mg (0,168 mmol) der Verbindung 4c wurden in 7 ml MeCN und 57,3 µl (0.521 mmol) NMM gelöst und auf 0 °C abgekühlt. Nach Zugabe von 46 mg (0,185 mmol) Cbz-OSu wurde der Ansatz 1 h bei 0°C und über Nacht bei RT gerührt. Das LM wurde i.V. entfernt und der Rückstand in ges. NaHCO₃-Lsg./EtAc aufgenommen. Die org. Phase wurde 3 x mit ges. NaHCO₃-Lsg. und 3 x mit ges. NaCl-Lsg. gewaschen, mit MgSO₄ getrocknet und das LM i.V. entfernt. Der gelbe Rückstand wurde mit je 5 ml EtOH und 1 N NaOH versetzt und 3 h bei RT gerührt. Nach Entfernen des LM i.V. wurde der Rückstand in 8 ml 30% Laufmittel B gelöst und mittels präp. HPLC gereinigt (Start bei 20% B). Die Produkt enthaltenen Fraktionen wurden vereint, das LM i.V. entfernt, der Rückstand in 40% t-Butanol/Wasser gelöst und lyophilisiert.
Ausbeute: 51 mg weißer, lyoph. Feststoff (HPLC: 25,5 min, Start bei 10 % B, MS: ber.: 670,31 gef.: 671,37 [M+H]⁺, 693,39 [M+Na]⁺).

### 5)

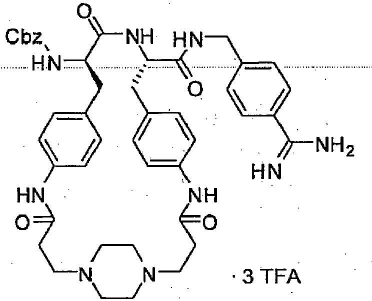

40 mg (0,0445 mmol) der Verbindung 5a wurden in 2 ml DMF gelöst und auf -15 °C gekühlt. Nach Zugabe von 14,7 µl (0,134 mmol) NMM und 5,8 µl (0,0445 mmol) CKIBE wurde die Lösung für 15 min gerührt und mit 14,8 mg (0,0668 mmol) 4-AMBA·2 HCl und 4,9 µl (0,0445 mmol) NMM versetzt. Der Ansatz wurde 1 h bei -15°C und über Nacht bei RT gerührt. Das LM wurde i.V. entfernt, der Rückstand in 8 ml 30% Laufmittel B gelöst und mittels präp. HPLC gereinigt (Start bei 10% B). Die Produkt enthaltenen Fraktionen wurden vereint, das LM i.V. entfernt, der Rückstand in 40% tert.-Butanol/Wasser gelöst und lyophilisiert.
Ausbeute: 21,1 mg weißer, lyoph. Feststoff (HPLC: 22,6 min, Start bei 10 % B, MS: ber.: 801,40 gef.: 802,50 [M+H]⁺, 401,81 [M+2H]⁺⁺/2).

### Beispiel 6: Synthese des Inhibitors 6

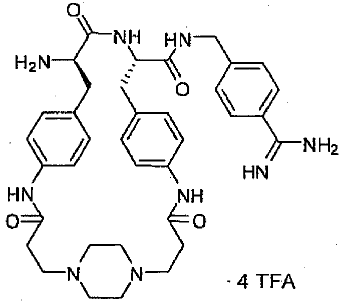

### 6)

10 mg (0,00874 mmol) der Verbindung 5 wurden mit 500 µl HBr/Eisessig versetzt und für 1 h, unter gelegentlichen Schwenken, stehen gelassen. Das Produkt wurde mit Diethylether gefällt, in 8 ml 20% Laufmittel B gelöst und mittels präpartiver HPLC (Start bei 0% B) gereinigt. Die Produkt enthaltenen Fraktionen wurden vereint, das LM i.V. entfernt, der Rückstand in 40% tert.-Butanol/Wasser gelöst und lyophilisiert.
Ausbeute: 8,2 mg weißer, lyoph. Feststoff (HPLC: 18,4 min, Start bei 1% B, MS: ber.: 667,36 gef.: 668,2 [M+H]⁺).

### Beispiel 7: Synthese des Inhibitors 7

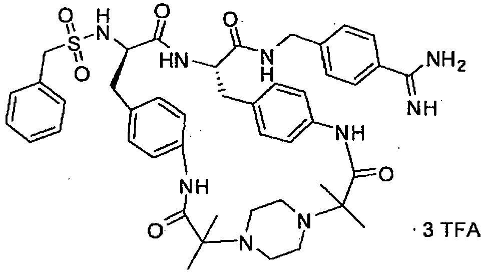

### 7a) Piperazin-N,N'-Di-(2,2-dimethyl)essigsäurebenzylester ·2 TFA

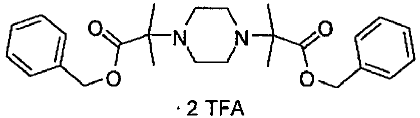

1,5 g (8,98 mmol) 2-Brom-2-Methylpropionsäure, 934 µl (8,98 mmol) Benzylalkohol und 15,8 mg (0,084 mmol) Toluolsulfonsäure-Monohydrat wurden mit 4,5 ml Toluol versetzt. Der Ansatz wurde für 18 h unter Rückfluß mittels eines Wasserabscheiders gekocht. Das LM wurde i.V. entfernt und das gelbe Öl in 5%-KHSO₄-Lsg./EtAc aufgenommen. Die org. Phase wurde 3 x mit 5%-KHSO₄-Lsg., 1 x mit ges. NaCl-Lsg., 3 x mit ges. NaHCO₃-Lsg. und 3 x mit ges. NaCl-Lsg. gewaschen, mit MgSO₄ getrocknet, filtriert und das LM i.V. entfernt (HPLC: 36,3 min, Start bei 30 % B).
1,39 g (5,41 mmol) des 2-Brom-2-Methyl-propionsäurebenzylesters und 212 mg (2,46 mmol) Piperazin wurden in 30 ml DMF gelöst. Der Ansatz wurde mit 1,43 g (6,15 mmol) Ag₂O versetzt und über Nacht bei RT gerührt. Nach HPLC-Kontrolle wurden weitere 0,715 g (3,075 mmol) Ag₂O hinzugefügt und der Ansatz nochmals über Nacht gerührt. Die Silbersalze wurden abfiltriert und das LM i.V. entfernt. Der Rückstand wurde gelöst und mittels präp. HPLC gereinigt (Start bei 30% B)
Ausbeute: 235,9 mg weißer, amorpher Feststoff (HPLC: 20,6 min, Start bei 30 % B, MS: ber.: 438,25 gef.: 439,10 [M+H]⁺).

### 7b) Piperazin-N,N'-Di-(2,2-dimethyl)essigsäure

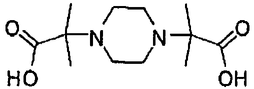

220 mg (0,330 mmol) Piperazin-N,N'-Di-(2,2-dimethyl)essigsäurebenzylester·2 TFA wurde in 80 ml 90% Essigsäure gelöst und mit 22 mg Pd/C versetzt. Nach dreimaligen sekurieren wurde der Ansatz über Nacht mit Wasserstoff hydriert. Die Suspension wurde filtriert und das LM i.V. entfernt. Der gelbe Rückstand wurde in wenig Methanol gelöst und mit Diethylether gefällt.
Ausbeute: 63,4 mg weiße, krist. Substanz (MS: ber.: 258, 16 gef.: 259,00 [M+H]⁺).

### 7c)

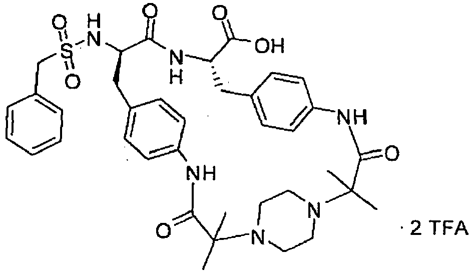

91 mg (0,178 mmol) Bzls-D-Phe(4-NH₂)-Phe(4-NH₂)-OMe und 43 mg (0,166 mmol) Piperazin-N,N'-Di-(2,2-dimethyl)essigsäure wurden in 75 ml DMF suspendiert und auf dem Eisbad gekühlt. Nach Hinzufügen von 165,3 mg (0,435 mmol) HATU und 182 µl (1,046 mmol) DIPEA wurde der Ansatz 1 h bei 0°C und anschließend 3 d bei RT gerührt. Nach Entfernen des LM i.V. wurde der Rückstand in je 3 ml EtOH und 1 N NaOH gelöst und 2,5 h bei RT gerührt. Der Ansatz wurde mit TFA neutralisiert und das LM i.V. entfernt. Der Rückstand wurde gelöst und mittels präp. HPLC gereinigt (Start bei 20% B).
Ausbeute: 17 mg weiße, lyoph. Substanz (HPLC: 30,3 min, Start bei 10 % B, MS: ber.: 718,31 gef.: 719,30 [M+H]⁺).

### 7)

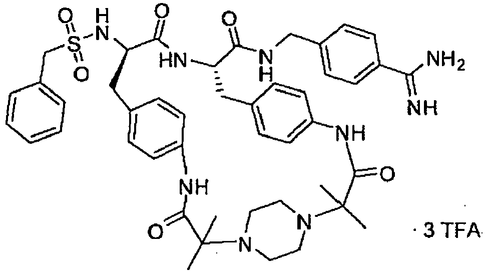

16 mg (0,0169 mmol) der Verbindung 7c wurde in 500 µl DMF gelöst und auf -15 °C abgekühlt. Nach Zugabe von 5,6 µl (0,0507 mmol) NMM und 2,2 µl (0,00169 mmol) CKIBE wurde die Lösung für 15 min gerührt und anschließend mit 5,6 mg (0,0250 mmol) 4-AMBA·2 HCl und 1,9 µl (0,0169 mmol) NMM versetzt. Der Ansatz wurde 1 h bei -15°C und über Nacht bei RT gerührt. Das LM wurde i.V. entfernt, der Rückstand in 8 ml 35% Laufmittel B gelöst und mittels präp. HPLC gereingt (Start bei 15% B). Die Produkt enthaltenen Fraktionen wurden vereint, das LM i.V. entfernt, der Rückstand in 40% t-Butanol/Wasser gelöst und lyophilisiert.
Ausbeute: 5,8 mg weiße, lyoph. Substanz (HPLC: 26,2 min, Start bei 10 % B, MS: ber.: 849,40 gef.: 850,50 [M+H]⁺).

### Beispiel 8: Synthese weiterer Verbindungen

Die folgenden Verbindungen wurden analog der Synthesestrategie für Verbindung 7 synthetisiert, nur das im Schritt a) an Stelle der 2-Brom-2-Methylpropionsäure die Verbindungen *R*-2-Brom-propionsäure, *S*-2-Brom-propionsäure oder *R*/*S*-2-Brom-propionsäure eingesetzt wurden.

### Inhibitor 8: (unter Verwendung von R-2-Brom-propionsäure im Schritt a)

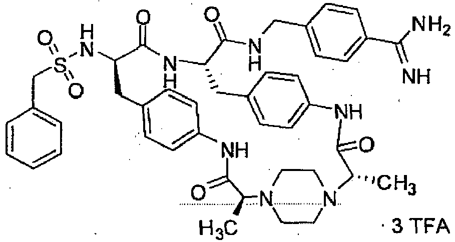

Ausbeute: 37,8 mg weißer, lyoph. Feststoff (HPLC: 23,4 min, Start bei 10 % B, MS: ber.: 821,37 gef.: 822,5 [M+H]⁺).

### Inhibitor 9: (unter Verwendung von S-2-Brom-propionsäure im Schritt a)

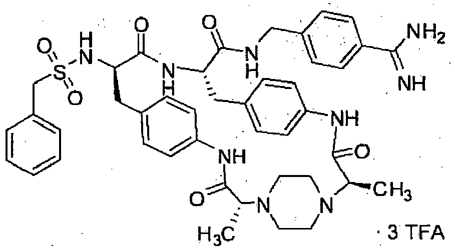

Ausbeute: 39,6 mg weißer, lyoph. Feststoff (HPLC: 23,6 min, Start bei 10 % B, MS: ber.: 821,37 gef.: 822,5 [M+H]⁺).

### Inhibitor 10: (Verwendung von racemischer 2-Brom-propionsäure im Schritt a)

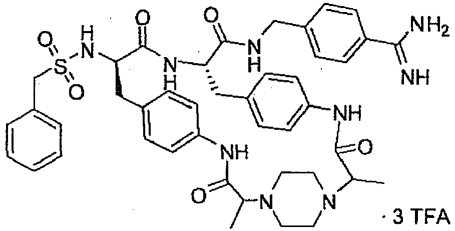

Ausbeute: 103,7 mg weißer, lyoph. Feststoff (HPLC: 21,1; 23,4; 23,6; 23,9 min, Start bei 10 % B, MS: ber.: 821,37 gef.: 822,5 [M+H]⁺).

### Inhibitor 11: (Die Synthese dieses Inhibitors erfolgte analog der Synthese des Inhibitors 5, wobei für die Zyklisierungsreaktion N,N'-Piperazindiessigsäure (Li Shen et al. ChemEurJ, 2006, 12, 4393-4396) eingesetzt wurde.)

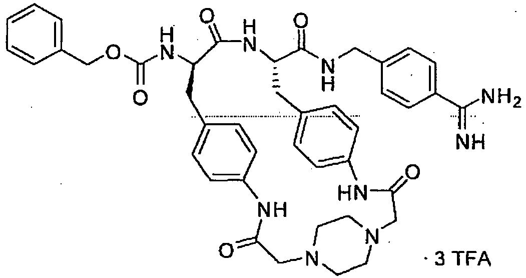

Ausbeute: 6 mg weißer, lyoph. Feststoff (HPLC: 21,68 min, Start bei 10 % B, MS: ber.: 773,36 gef.: 774,3 [M+H]⁺).

### Inhibitor 12: (Die Synthese dieses Inhibitors erfolgte durch Abspaltung der Cbz-Schutzgruppe des nachsynthetisierten Inhibitors 11 mit 32% HBr in Essigsäure (1 h bei Raumtemperatur).

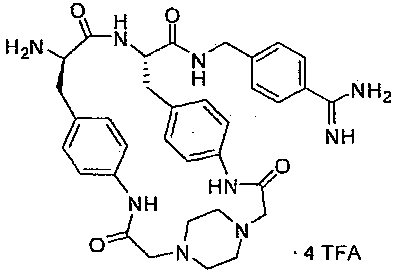

Ausbeute: 14 mg weißer, lyoph. Feststoff (HPLC: 19,09 min, Start bei 1 % B, MS: ber.: 639,33 gef.: 320,86 [M+2H]²⁺/2).

**Inhibitor 13:** (Die Synthese dieses Inhibitors erfolgte durch Kopplung von Boc-Diaminobutan an Intermediat 1e mittels PyBOP/DIPEA in DMF, anschließender Abspaltung der Boc-Schutzgruppe mit Trifluoressigsäure und Aufbau der Guanidinogruppe durch Umsetzung mit jeweils 3 Equivalenten 1*H*-pyrazole-1-carboxamidine· HCl und DIPEA in DMF bei Raumtemperatur über Nacht sowie finaler präparativer HPLC.)

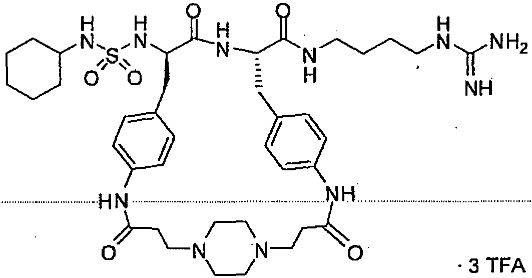

Ausbeute: 12 mg weißer, lyoph. Feststoff (HPLC: 21,0 min, Start bei 10 % B, MS: ber.: 809,44 gef.: 810,47 [M+H]⁺).

**Inhibitor 14:** (Die Synthese dieses Inhibitors erfolgte durch Kopplung von N,N-Dimethylsulfamoylchlorid (Merck) an ein zu Verbindung 4c analoges Intermediat, das jedoch mit N,N'-Piperazindiessigsäure zyklisiert wurde. Nach Verseifung des Methlesters und Ankopplung des 4-Amidinobenzylamids wurde der finale Inhibitor erhalten und mittels präparativer HPLC gereinigt).

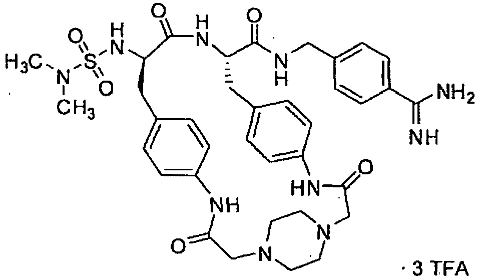

Ausbeute: 16 mg weißer, lyoph. Feststoff (HPLC: 14,22 min, Start bei 10 % B, MS: ber.: 746,33 gef.: 747,67 [M+H]⁺).

**Inhibitor 15:** (Die Synthese dieses Inhibitors erfolgte durch Kopplung von N,N-Dimethylsulfamoylchlorid (Merck) an Verbindung 4c. Nach Verseifung des Methylesters und Ankopplung des 4-Amidinobenzylamids wurde der finale Inhibitor erhalten und mittels präparativer HPLC gereinigt). 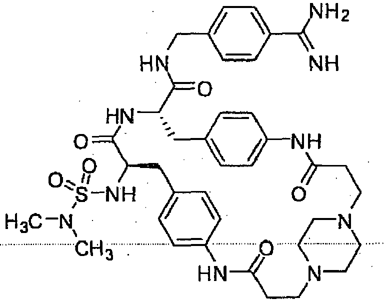 weißer, lyoph. Feststoff (HPLC: 14,9 min, Start bei 10 % B).

**Inhibitor 16:** (Die Synthese dieses Inhibitors erfolgte analog der Synthese des Inhibitors 4, wobei an Stelle der Phenylessigsäure in diesem Fall Phenylpropionsäure mittels des Mischanhydridverfahrens an Intermediat 4c gekoppelt wurde.) 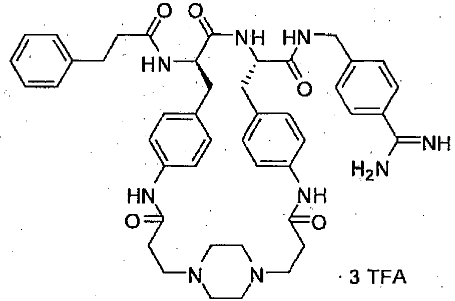 weißer, lyoph. Feststoff (HPLC: 20,2 min, Start bei 10 % B).

**Inhibitor 17:** (Die Synthese dieses Inhibitors erfolgte analog der Synthese des Inhibitors 4, wobei an Stelle der Phenylessigsäure in diesem Fall Phenylbuttersäure mittels des Mischanhydridverfahrens an Intermediat 4c gekoppelt wurde.) 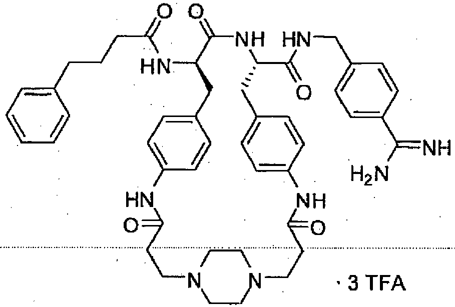 weißer, lyoph. Feststoff (HPLC: 21,0 min, Start bei 10 % B).

### Beispiel 9: Enzymkinetische Messungen

Die Bestimmung der Hemmkonstanten für Plasmin, PK, Thrombin und Faktor Xa erfolgte analog beschriebener Methoden (Stürzebecher et al., 1997) in einem Microplate Reader (Multiscan Ascent der Firma Thermo) bei 405 nm. Für die Bestimmungen wurden die in Tabelle 1 zusammengefassten Enzyme und Substrate verwendet.

**Tabelle 1: Verwendete Enzyme und Substrate**

| Enzym | Substrat |
|---|---|
| Plasmin (human), Chromogenix, Spez. Aktivität: 11 CU/mg | Tos-Gly-Pro-Lys-pNA (Chromozym PL) |
| | 4 mM (364 µM im Ansatz) |
| | 2 mM (182 µM im Ansatz) |
| | 1 mM (91 µM im Ansatz) |
| Plasma-Kallikrein (human), Enzyme Research South Bend | H-D-Pro-Phe-Arg-pNA (Haemochrom PK) |
| | 2 mM (182 µM im Ansatz) |
| | 1 mM (91 µM im Ansatz) |
| | 0,5 mM (45,5 µM im Ansatz) |
| Thrombin (Rind), 1425 IE/mg | CH₃SO₂-D-Cha-Gly-Arg-pNA (Pefachrome tPA) |
| | 2 mM (182 µM im Ansatz) |
| | 1 mM (91 µM im Ansatz) |
| | 0,5 mM (45,5 µM im Ansatz) |
| Faktor Xa (human) 2530PL, 200.35 IE/mg, | CH₃OCO-D-CHA-Gly-Arg-pNA (Pefachrome FXa) |
| Enzyme Research South Bend | 2 mM (182 µM im Ansatz) |
| | 1 mM (91 µM im Ansatz) |
| | 0,5 mM (45,5 µM im Ansatz) |

### Messansatz:

- 200 µl 50 mM Tris x HCl Puffer pH 8.0 (enthält 0,154 M NaCl, 2 % Ethanol und den Inhibitor in geeigneter Konzentration).
- 25 µl Substrat
- Start mit 50 µl Enzymlösung

Die Messungen wurden durch Zugabe von 25 µl 50 % Essigsäure abgestoppt und die Kᵢ-Werte nach Dixon berechnet Die Kᵢ-Werte sind das Mittel aus mindestens zwei Bestimmungen.

### Hemmwirkung:

Die Inhibitoren 1-10 hemmen Plasmin mit Kᵢ-Werten < 15 nM, während Thrombin, Plasmakallikrein und Faktor Xa mit Hemmkonstanten > 50 nM inhibiert werden.

Exemplarisch sind in der folgenden Tabelle 2 für einige ausgewählte Inhibitoren die konkreten Hemmkonstanten angegeben.

**Tabelle 2: Hemmung der trypsinartigen Serinproteasen Plasmin, Thrombin, Faktor Xa (FXa) und Plasmakallikrein (PK) durch ausgewählte Inhibitoren (Kᵢ in nM, n.b. = nicht bestimmt).**

| Inhibitor | Kᵢ (nM) | | | |
|---|---|---|---|---|
| | Plasmin | PK | Thrombin | FXa |
| 1 | 0,052 | 600 | 17000 | 7400 |
| 2 | 0,32 | 319 | 16000 | 7900 |
| 3 | 1,24 | 6380 | 141000 | 78000 |
| 4 | 8,0 | 7920 | 41800 | 188400 |
| 5 | 0,77 | 6950 | 44000 | 47400 |
| 6 | 12,8 | 24500 | 60800 | 404500 |
| 7 | 2,2 | 77 | 620 | 1450 |
| 11 | 5,4 | 4640 | 46900 | 104200 |
| 13 | 3,5 | n.b. | n.b | n.b. |
| 14 | 5,9 | 12000 | 4960 | 134400 |

## Patentansprüche

1. Verbindung gemäß Formel (I) 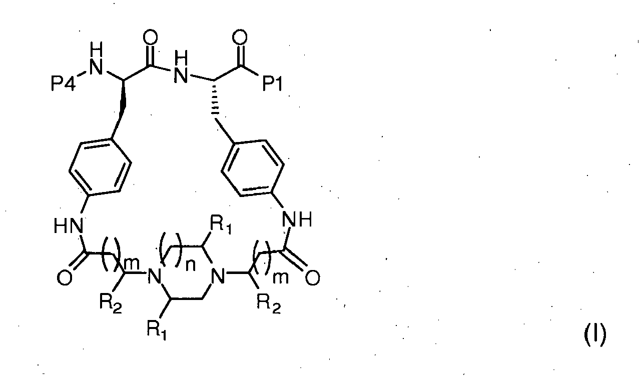 oder ein Salz dieser Verbindung, **dadurch gekennzeichnet, dass**
- n gleich 1 oder 2 ist, und
- m gleich 0, 1 oder 2 ist, und
- R₁ entweder H oder ein verzweigtes oder unverzweigtes Alkyl mit bis zu 3 Kohlenstoffatomen ist, und
- R₂ entweder H oder ein verzweigtes oder unverzweigtes Alkyl mit bis zu 5 Kohlenstoffatomen oder ein Aryl- bzw. Aralkylrest mit bis zu 7 Kohlenstoffatomen ist, und
- P1 einer der folgenden Reste 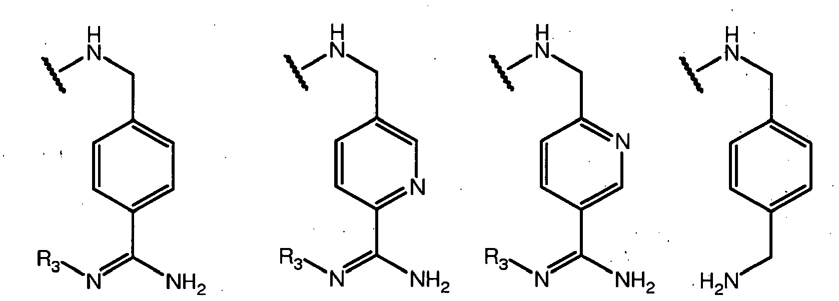 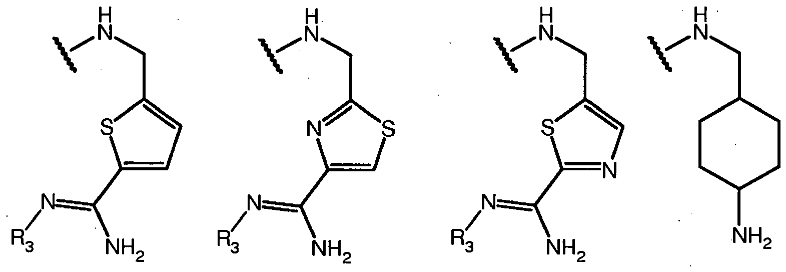 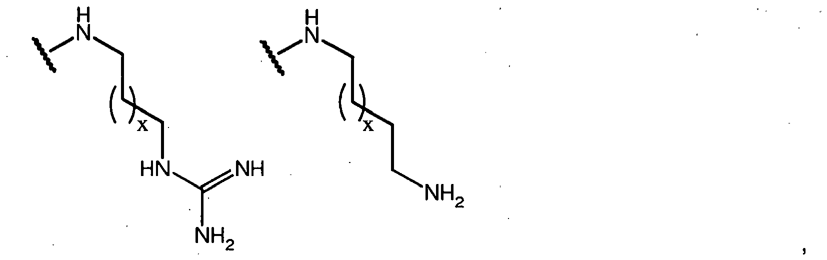 ist, wobei R₃ ein H, OH, O-CH₃, NH₂, O-CO-CH₃ oder CO-O-(CH₂)_{z}-CH₃ sein kann und z eine ganze Zahl von 1 bis 5 ist, und x eine ganze Zahl von 1 bis 3 ist, und
- P4 entweder ein H, SO₂-R₄, SO₂-NH₂, SO₂-NH-R₄, SO₂-N(R₄)₂, CO-O-R₄, CO-R₄, CH₂-COOH oder ein CH₂-COOEt ist, wobei R₄ ein verzweigter oder unverzweigter oder zyklischer Alkylrest mit 1 bis 10 Kohlenstoffatomen, oder ein Aryl-, ein Heteroaralkyl- oder Aralkylrest mit 6 bis 10 Kohlenstoffatomen sein kann, wobei der Heteroaralkylrest 1 bis 3 Heteroatome ausgewählt aus N, S oder O enthalten kann, und wobei der genannte Alkyl-, Aryl-, Aralkyl- und Heteroaralkylrest gegebenenfalls mit 1 bis 2 Resten an beliebiger Stelle substituiert sein kann, die ausgewählt werden aus Aminomethylen, Cyano, CF₃, Tetrazol, F, Cl, Br, COOH, COOEt, COOMe, Methoxy, Ethoxy, Isopropoxy, Methyl, Ethyl, oder Isopropyl, und
wobei die Verbindung gemäß Formel (I) nicht 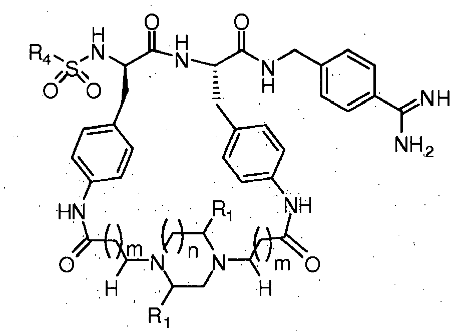 mit R₁, R₄, m und n wie oben definiert ist.

2. Verbindung nach Anspruch 1, **dadurch gekennzeichnet, dass** R₁ ein H ist.

3. Verbindung nach Ansprüchen 1 bis 2, **dadurch gekennzeichnet, dass** R₂ ein H ist.

4. Verbindung nach Ansprüchen 1 bis 3, **dadurch gekennzeichnet, dass** P4 ein H, SO₂-NH₂ oder eine der folgenden Strukturen 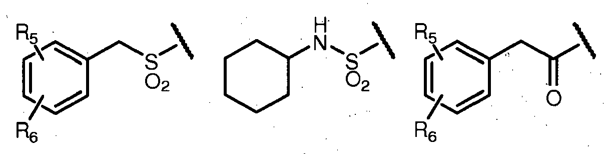 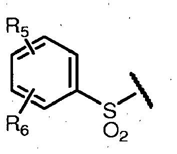 ist, wobei R₅ und R₆ unabhängig voneinander entweder H oder Aminomethylen, Cyano, CF₃, Tetrazol, F, Cl, Br, COOH, COOEt, COOMe, Methoxy, Ethoxy, Isopropoxy, Methyl, Ethyl, oder Isopropyl sind, bevorzugt sind R₅ und R₆ ein H.

5. Verbindung nach Ansprüchen 1 bis 4, **dadurch gekennzeichnet, dass** P1 aus folgenden Strukturen ausgewählt wird 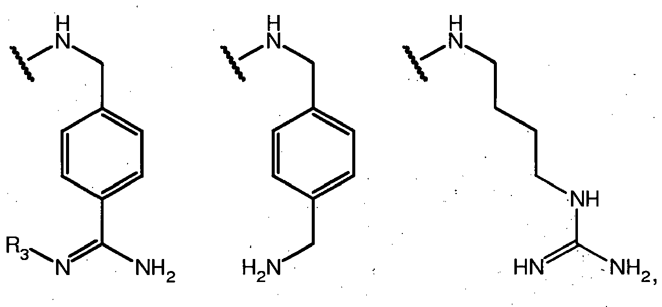 wobei R₃ definiert ist wie zuvor, aber bevorzugt ein H ist.

6. Verbindung nach Ansprüchen 1 bis 5, **dadurch gekennzeichnet, dass** m gleich 0 oder 1 ist.

7. Verbindung nach Ansprüchen 1 bis 6, **dadurch gekennzeichnet, dass** sie folgende Strukturen besitzen können 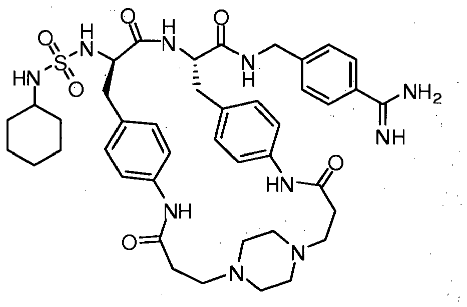 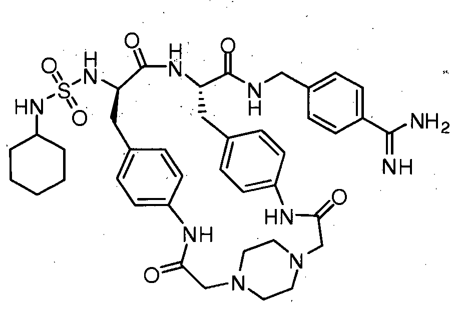 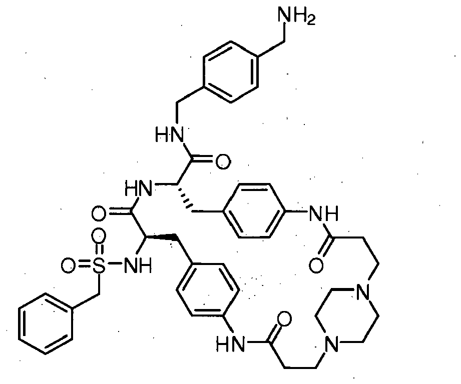 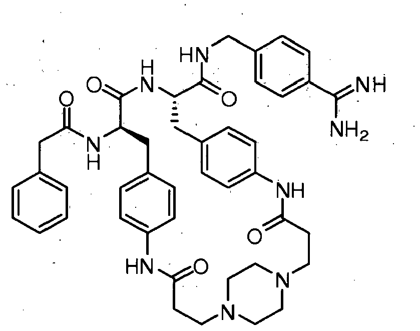 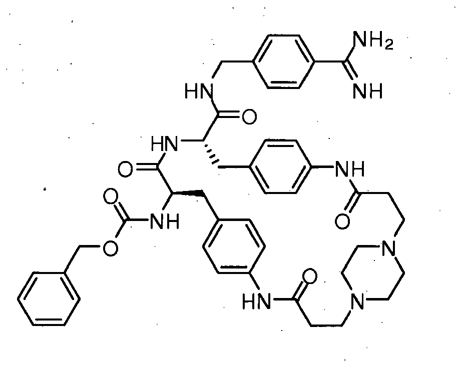 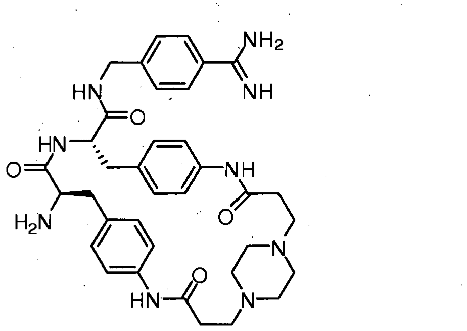 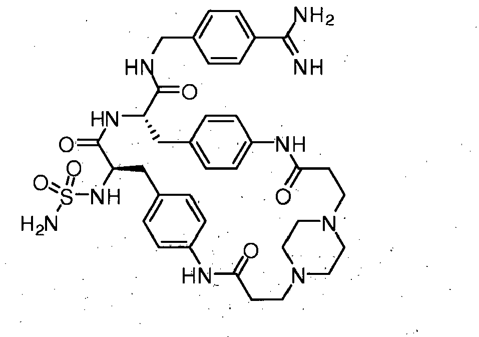 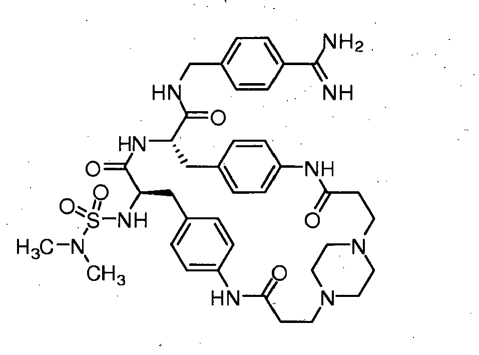 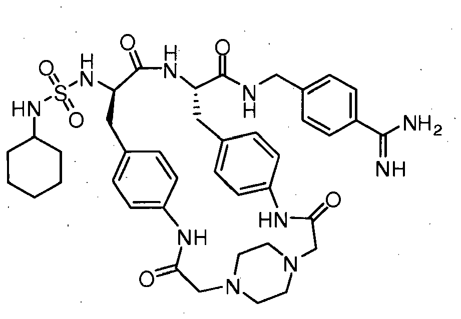 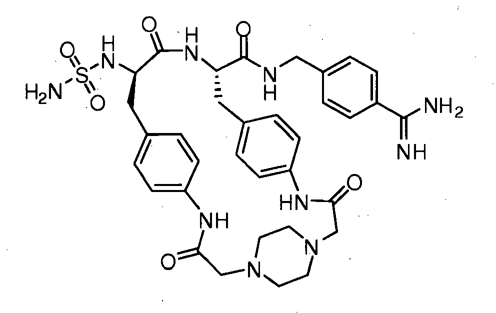 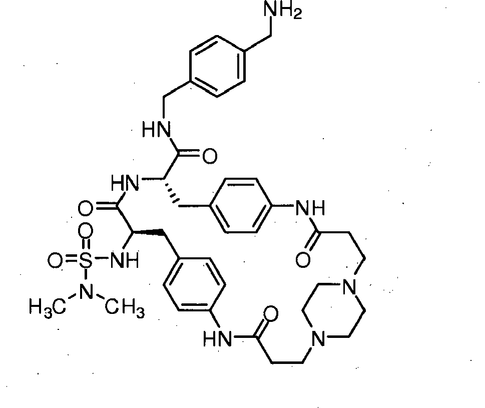 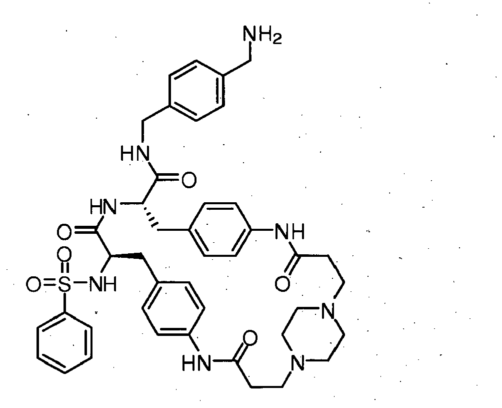 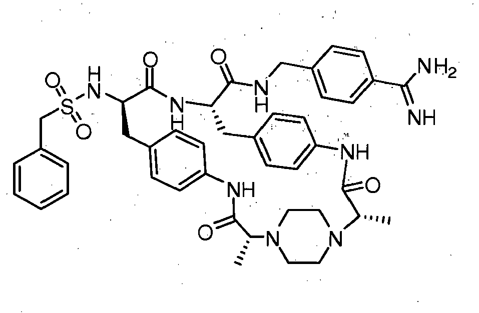 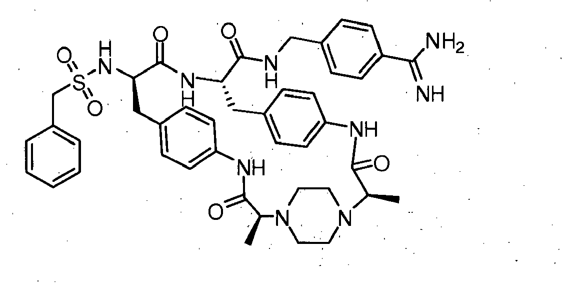 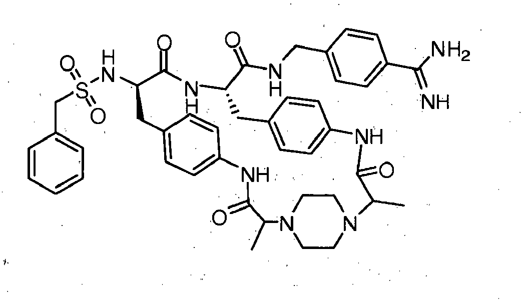 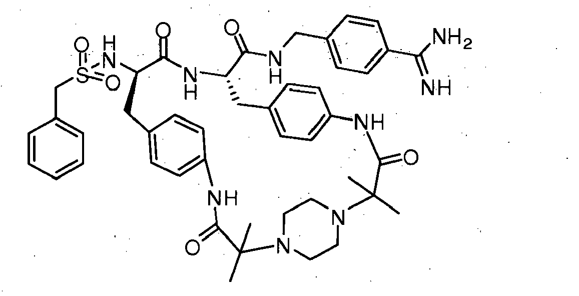 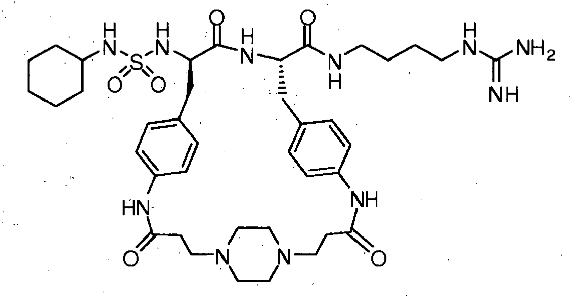 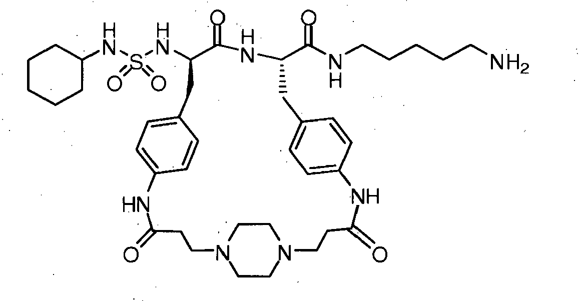 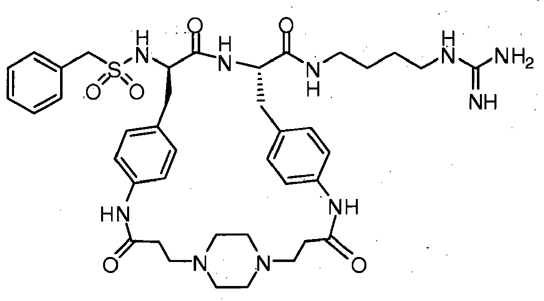 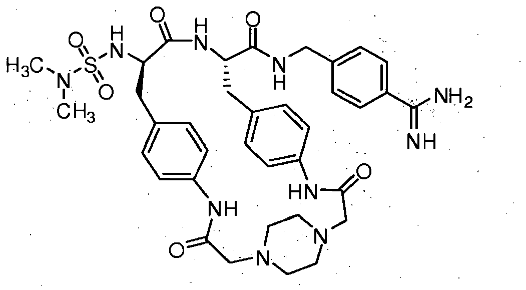 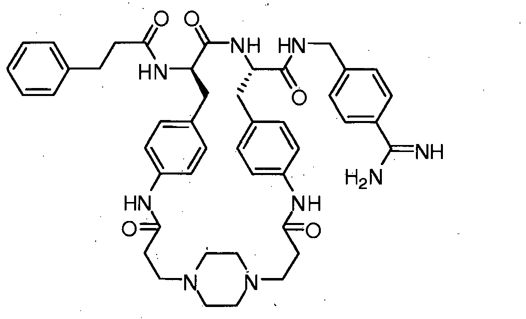 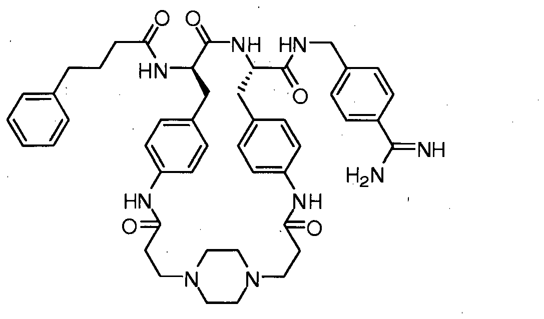 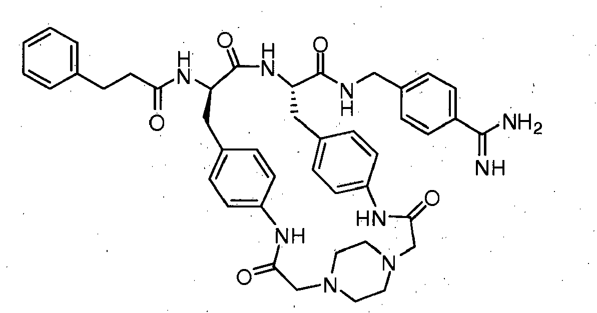 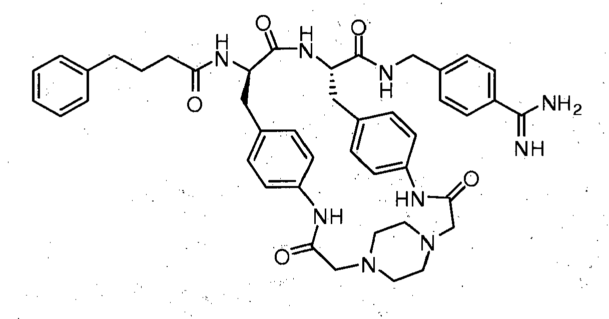 wobei die basischen und gegebenenfalls vorhandenen sauren Gruppen in einer physiologisch verträglichen Salzform vorliegen.

8. Arzneimittel, enthaltend mindestens eine Verbindung gemäß einem der Ansprüche 1 bis 7.

9. Arzneimittel nach Anspruch 8, **dadurch gekennzeichnet, dass** es in Form einer Tablette, eines Dragees, einer Kapsel, eines Pellets, eines Suppositoriums, einer Lösung, insbesondere einer Injektions- oder Infusionslösung, von Augen-, Nasen- oder Ohrentropfen, eines Saftes, einer Emulsion oder Suspension, eines Globuli, eines Styli, eines Aerosols, eines Aerosol-Sprays, eines Puders, einer Paste, einer Creme oder einer Salbe eingesetzt wird, bevorzugt wird es als Injektions- oder Infusionslösung verwendet.

10. Verbindungen nach Anspruch 1 bis 7 und des Arzneimittels nach Anspruch 8 und 9 zur Verwendung, um den Blutverlust bei hyperfibrinolytischen Zuständen zu reduzieren.

11. Verbindungen nach Anspruch 1 bis 7 und des Arzneimittels nach Anspruch 8 und 9 zur Vermeidung von Blutverlusten bei chirurgischen Eingriffen, besonders bei Herzoperationen und Organtransplantationen, aber auch zur Linderung von starken Regelblutungen, zur Verhinderung oder Linderung von Blutungen bei Zahnextraktion oder Zahnfleischblutungen, vor allem bei Patienten mit Hämophilie, aber auch zur Therapie leichter Formen der Hämophilie.

12. Verwendung der Verbindungen nach Anspruch 1 bis 7 und des Arzneimittels nach Anspruch 8 und 9 als Mittel zur Herstellung eines Fibrinklebers.

13. Verbindungen nach Anspruch 1 bis 7 und des Arzneimittels nach Anspruch 8 und 9 zur Behandlung von Krebs oder der Metastasierung, zur Behandlung von Arthritis oder der proliferativen Vitreoretionpathie.

14. Verbindungen nach Anspruch 1 bis 7 und des Arzneimittels nach Anspruch 8 und 9 zur Verwendung als Antidot bei Thrombolysetherapien mit Plasmin oder Plasminaktivatoren, insbesondere nach Herzinfarkten, falls es zu Blutungskomplikationen kommt.

15. Verwendung der Verbindungen nach Anspruch 1 bis 7 für in vitro Anwendungen zur Hemmung des Plasmins, einschließlich für diagnostische Zwecke.

## Claims

1. Compound according to formula (I) 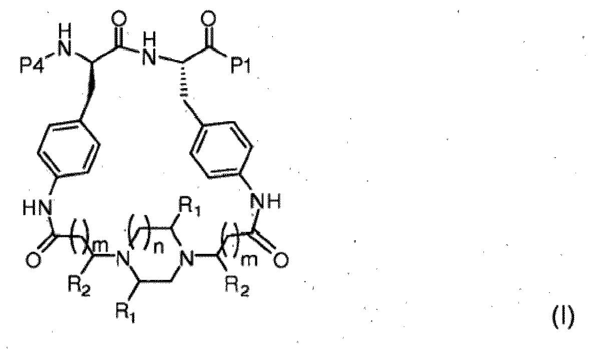 or a salt of said compound, **characterised in that**
- n equals 1 or 2, and
- m equals 0, 1 or 2, and
- R₁ is either H or a branched or unbranched alkyl having up to 3 carbon atoms,
and
- R₂ is either H or a branched or unbranched alkyl having up to 5 carbon atoms or an aryl- or aralkyl group having up to 7 carbon atoms, and
- P1 is one of the following groups: 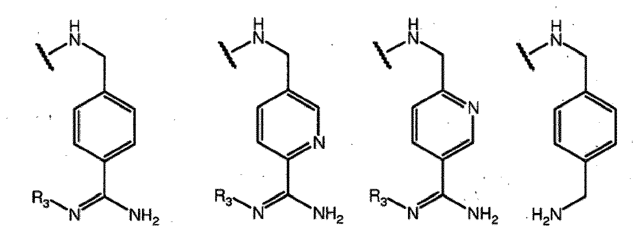 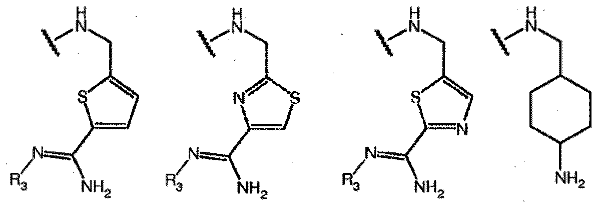 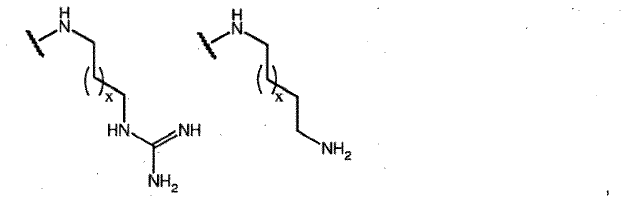
in which R₃ can be H, OH, O-CH₃, NH₂, O-CO-CH₃ or CO-O-(CH₂)_{z}-CH₃ and z is an integer from 1 to 5, and x is an integer from 1 to 3, and
- P4 is either H, SO₂-R₄, SO₂-NH₂, SO₂-NH-R₄, SO₂-N(R₄)₂, CO-O-R₄, CO-R₄, CH₂-COOH, or CH₂-COOEt, in which R₄ can be a branched or unbranched or cyclic alkyl group having 1 to 10 carbon atoms, or an aryl, a heteroaralkyl or aralkyl group having 6 to 10 carbon atoms, in which the heteroaralkyl group may contain 1 to 3 heteroatoms selected from N, S, or O, and in which the heteroaralkyl group can, if applicable, be substituted with 1 to 2 groups in an arbitrary position which are selected from the group comprising aminomethylene, cyano, CF₃, tetrazole, F, Cl, Br, COOH, COOEt, COOMe, methoxy, ethoxy, isopropoxy, methyl, ethyl, or isopropyl, and
in which the compound according to formula (I) is not 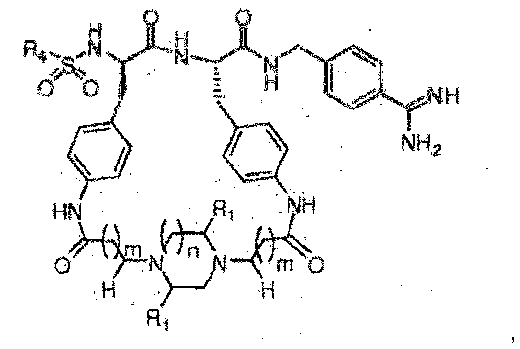 R₁, R₄, m and n being defined as above.

2. Compound according to claim 1, **characterised in that** R₁ is H.

3. Compound according to claims 1 and 2, **characterised in that** R₂ is H.

4. Compound according to claims 1 to 3, **characterised in that** P4 is H, SO₂-NH₂ or one of the following structures, 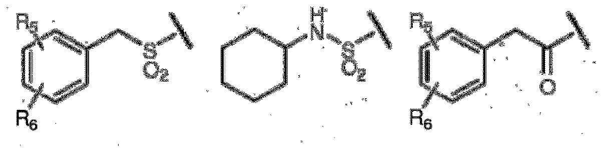 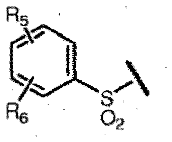 in which R₅ and R₆ are, independently of one another, either H or aminomethylene, cyano, CF₃, tetrazole, F, Cl, Br, COOH, COOEt, COOMe, methoxy, ethoxy, isopropoxy, methyl, ethyl, or isopropyl, preferably R₅ and R₆ are H.

5. Compound according to claims 1 to 4, **characterised in that** P1 is selected from the following structures, 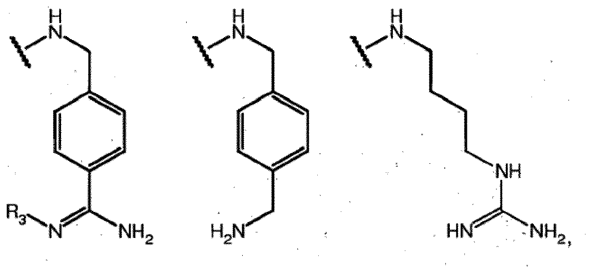 in which R₃ is defined as before, but is preferably H.

6. Compound according to claims 1 to 5, **characterised in that** m equals 0 or 1.

7. Compound according to claims 1 to 6, **characterised in that** it can have the following structures 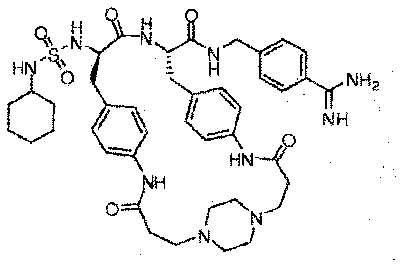 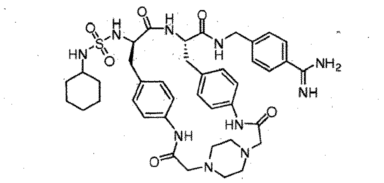 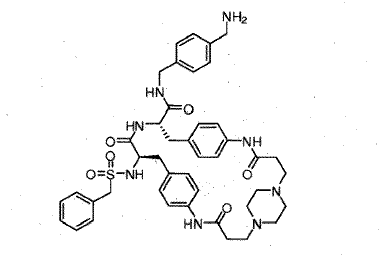 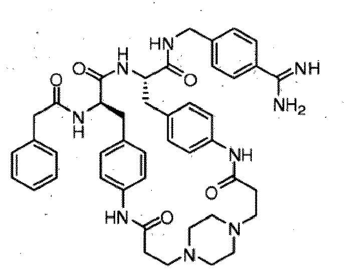 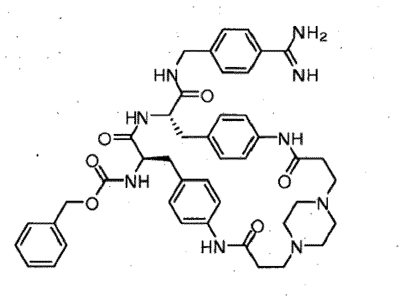 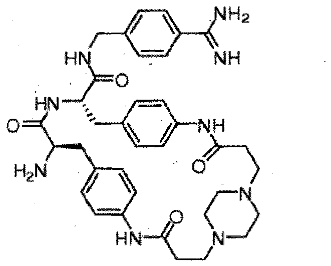 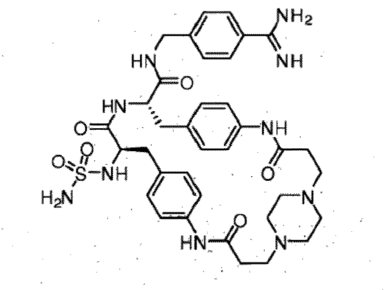 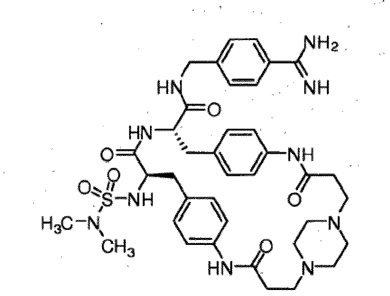 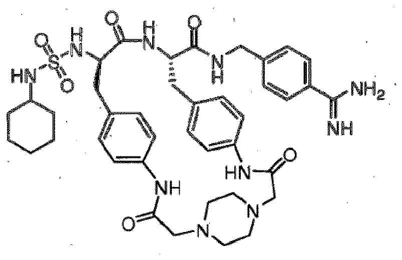 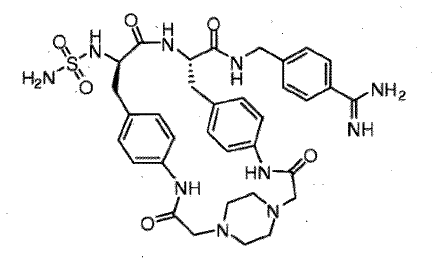 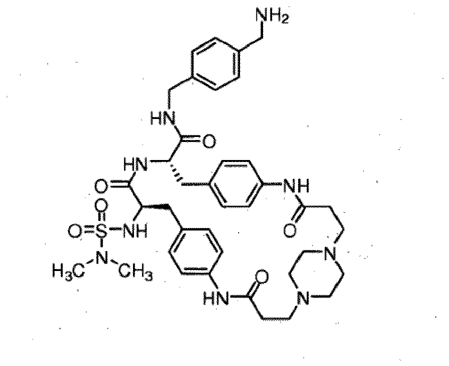 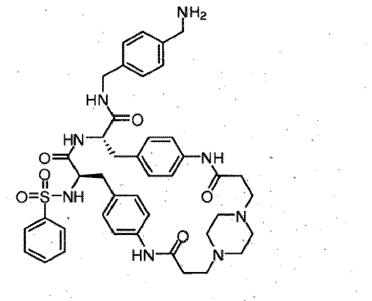 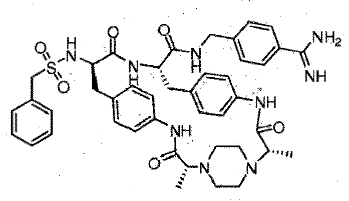 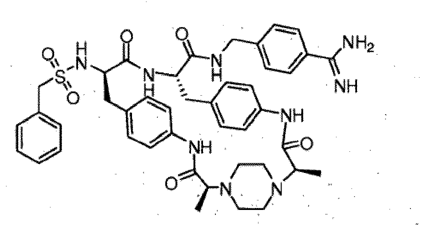 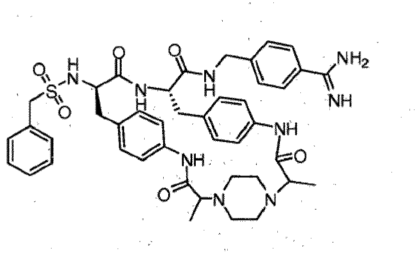 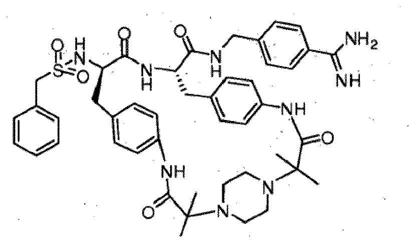 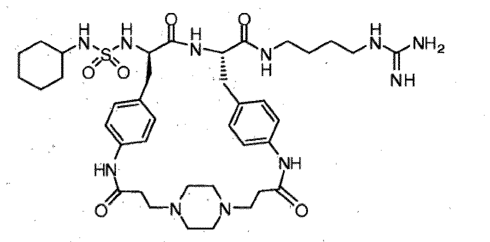 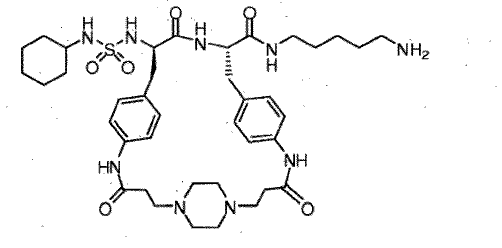 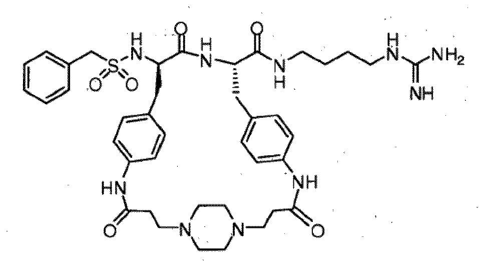 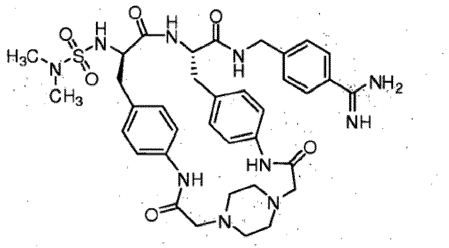 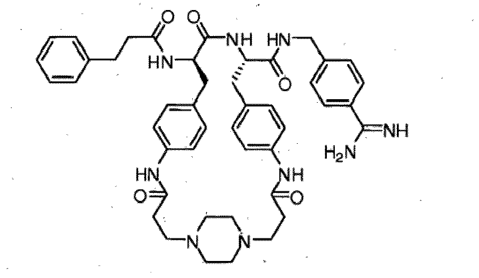 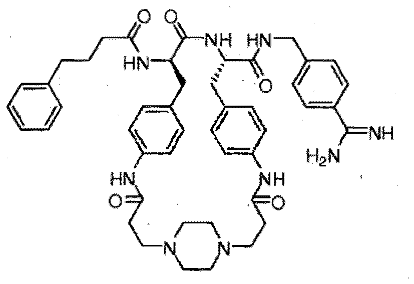 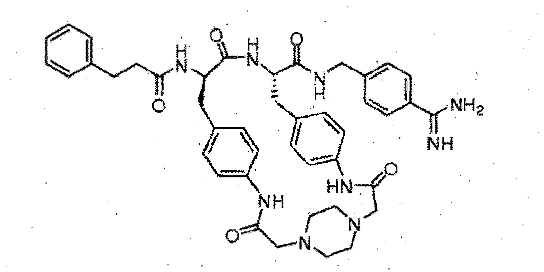 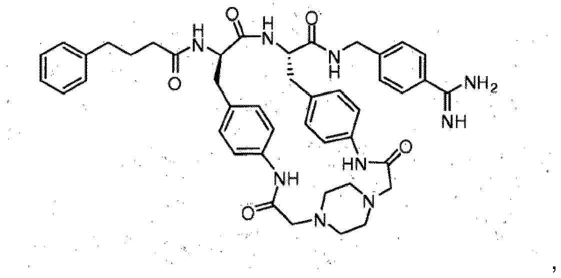 in which the basic and, if applicable, existing acidic groups are present in a physiologically acceptable salt form.

8. Pharmaceutical composition, comprising at least one compound according to any of claims 1 to 7.

9. Pharmaceutical composition according to claim 8, **characterised in that** it is used in the form of a tablet, a dragée, a capsule, a pellet, a suppository, a solution, in particular an injection or infusion solution, of eye, nose or ear drops, a juice, an emulsion or suspension, a globule, a styli, an aerosol, an aerosol spray, a powder, a paste, a cream or an ointment, preferably it is used as injection or infusion solution.

10. Compounds according to claims 1 to 7 and the pharmaceutical composition according to claim 8 and claim 9 to be used for reducing blood loss in the case of hyperfibrinolytic conditions.

11. Compounds according to claims 1 to 7 and the pharmaceutical composition according to claim 8 and claim 9 for preventing blood loss during surgical procedures, in particular during heart surgery and organ transplants, but also for alleviating severe menstrual bleeding, preventing or alleviating bleeding during tooth extraction or bleeding gums, particularly in patients with haemophilia, but also for treating mild forms of haemophilia.

12. Use of the compounds according to claims 1 to 7 and the pharmaceutical composition according to claim 8 and claim 9 as an agent for the preparation of fibrin adhesives.

13. Compounds according to claims 1 to 7 and the pharmaceutical composition according to claim 8 and claim 9 for treating cancer or metastasis, for treating arthritis or proliferative vitreoretinopathy.

14. Compounds according to claims 1 to 7 and the pharmaceutical composition according to claim 8 and claim 9 to be used as an antidote during thrombolytic therapy using plasmin or plasmin activators, in particular after heart attacks, if bleeding complications occur.

15. Use of the compounds according to claims 1 to 7 for in vitro applications for the inhibition of plasmin, including for diagnostic purposes.

## Revendications

1. Composé de formule (I) : 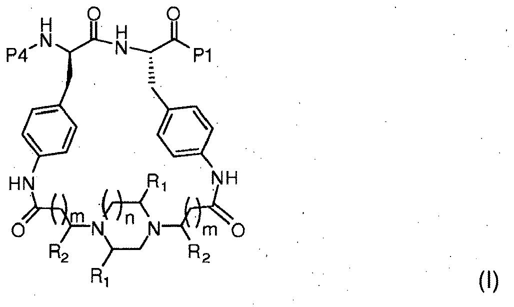 ou sel de ce composé, **caractérisé en ce que** :
- n est égal à 1 ou à 2,
- m est égal à 0, 1 ou 2,
- R₁ est H ou un groupement alkyle ramifié et non ramifié ayant jusqu'à 3 atomes de carbone,
- R₂ est H ou un groupement alkyle ramifié ou non ramifié ayant jusqu'à 5 atomes de carbone ou un groupement aryle ou aralkyle ayant jusqu'à 7 atomes de carbone, et
- P₁ est l'un des groupements suivants : 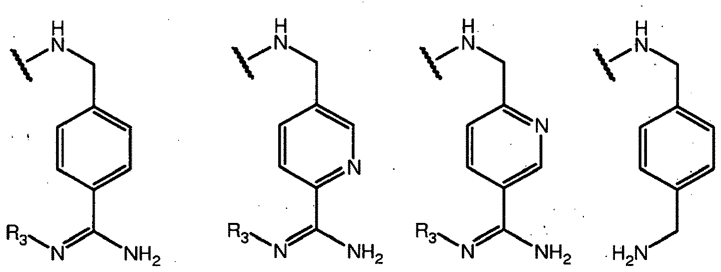 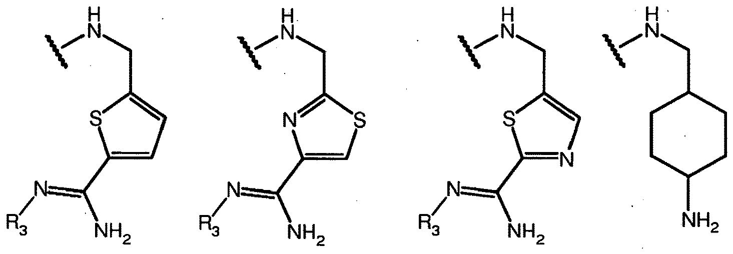 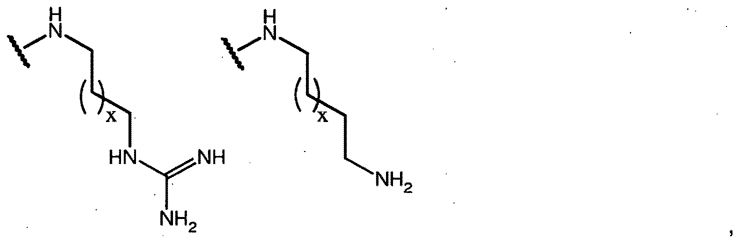
dans lequel R₃ peut être H, OH, O-CH₃, NH₂, O-CO-CH₃ ou CO-O-(CH₂)_{z} et z est un nombre entier de 1 à 5 et x un nombre entier de 1 à 3, et
- P₄ est H, SO₂-R₄, SO₂-NH₂, SO₂-NH-R₄, SO₂-N(R₄)₂, CO-O-R₄, CO-R₄, CH₂-COOH ou CH₂-COOEt, dans lequel R₄ peut être un groupement alkyle ramifié ou non ramifié ou cyclique ayant 1 à 10 atomes de carbone ou un groupement aryle, hétéroaralkyle ou aralkyle ayant 6 à 10 atomes de carbone, dans lequel le groupement hétéroaralkyle peut contenir 1 à 3 hétéroatomes choisis parmi N, S ou 0, et dans lequel les groupements alkyle, aryle, aralkyle et hétéroaralkyle mentionnés peuvent être éventuellement substitués par 1 à 2 groupements en un point quelconque, lesquels groupements sont choisis parmi les groupements aminométhylène, cyano, CF₃, tétrazole, F, Cl, Br, COOH, COOEt, COOMe, méthoxy, éthoxy, isopropoxy, méthyle, éthyle ou isopropyle, et
dans lequel le composé de formule (I) : 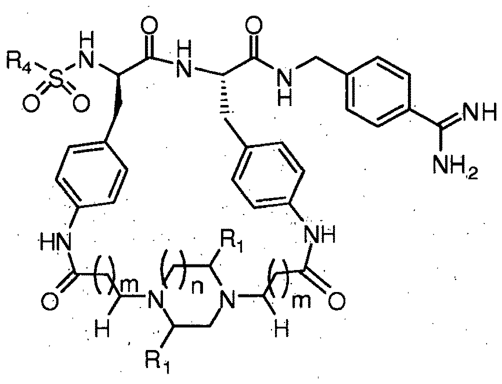 n'est pas défini avec R₁, R₄, m et n comme ci-dessus.

2. Composé selon la revendication 1, **caractérisé en ce que** R₁ est H.

3. Composé selon les revendications 1 à 2, **caractérisé en ce que** R₂ est H.

4. Composé selon les revendications 1 à 3, **caractérisé en ce que** P₄ est H, SO₂-NH₂ ou l'une des structures suivants : 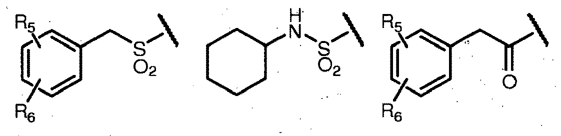 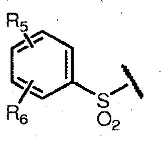 dans lequel R₅ et R₆ sont indépendamment l'un de l'autre H ou un groupement aminométhylène, cyano, CF₃, tétrazole, F, Cl, Br, COOH, COOEt, COOMe, méthoxy, éthoxy, isopropoxy, méthyle, éthyle ou isopropyle, de préférence R₅ et R₆ sont H.

5. Composé selon les revendications 1 à 4, **caractérisé en ce que** P₁ est choisi parmi les structures suivantes : 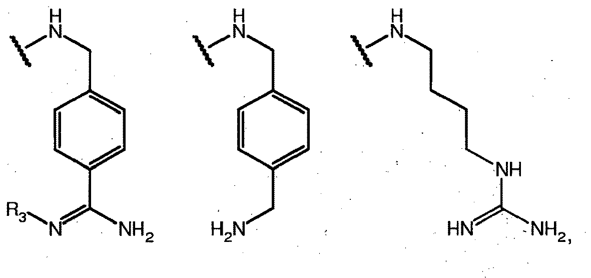 dans lequel R₃ est défini comme précédemment, mais est de préférence H.

6. Composé selon les revendications 1 à 5, **caractérisé en ce que** m est égal à 0 ou à 1.

7. Composé selon les revendications 1 à 6, **caractérisé en ce que** les composés peuvent posséder les structures suivantes : 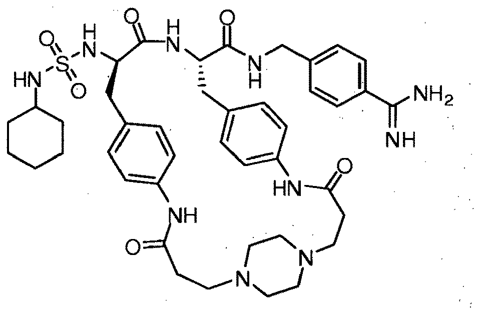 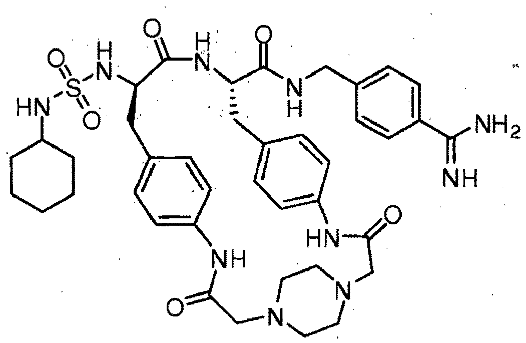 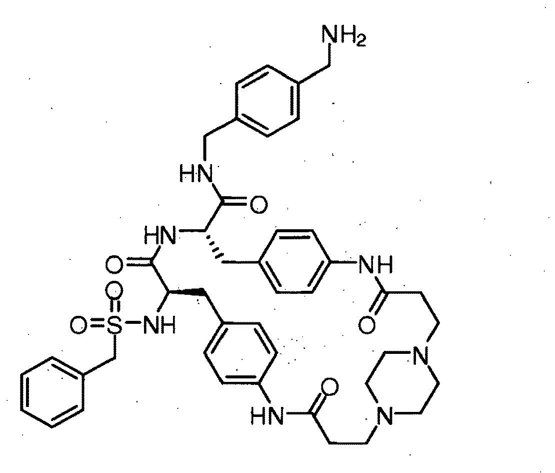 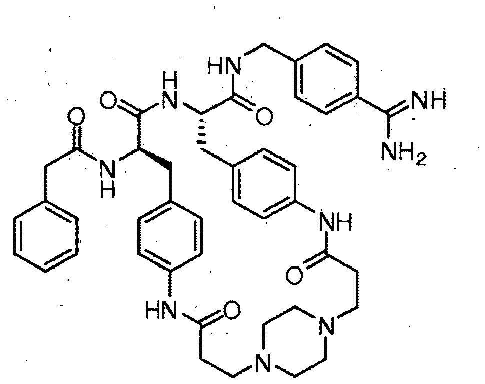 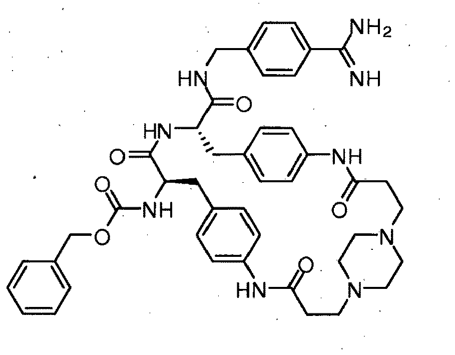 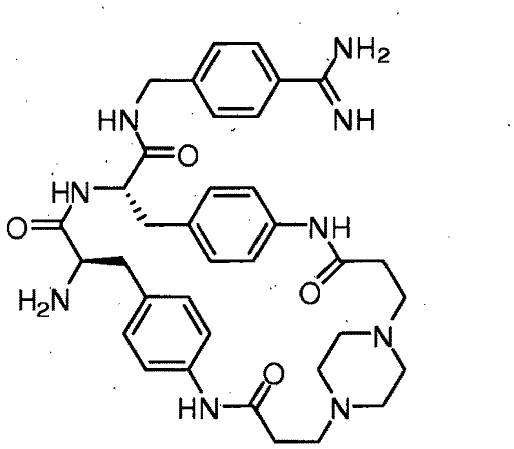 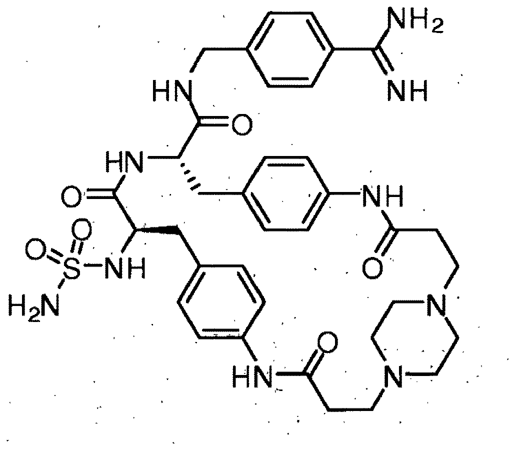 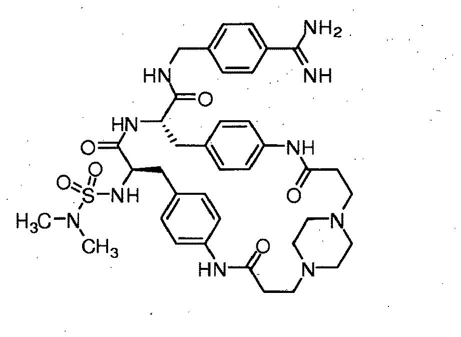 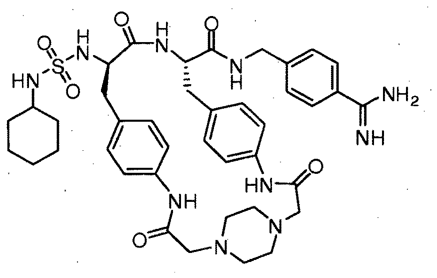 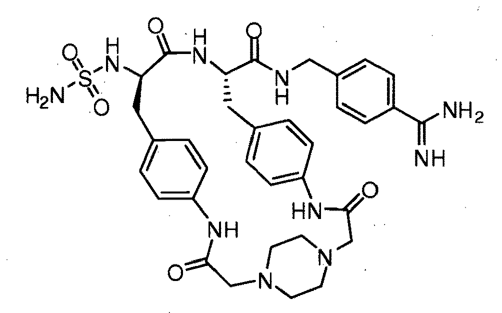 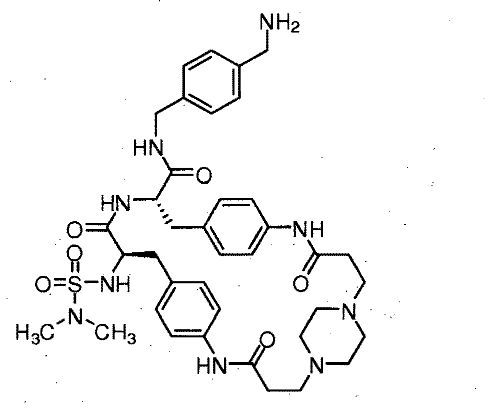 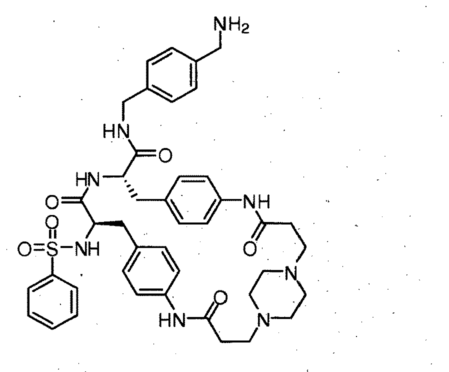 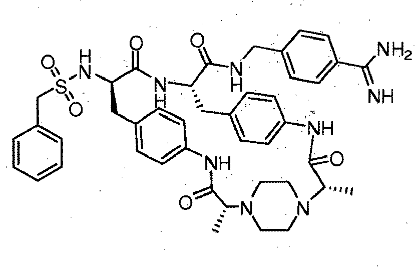 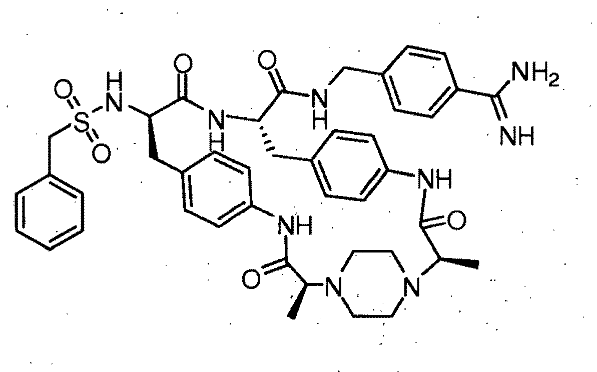 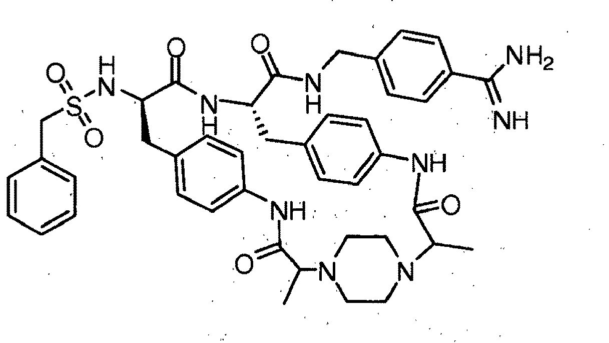 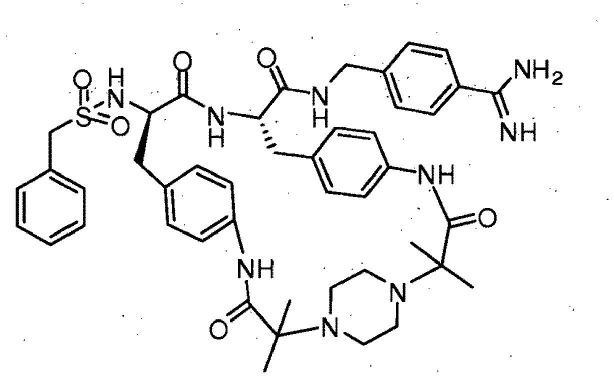 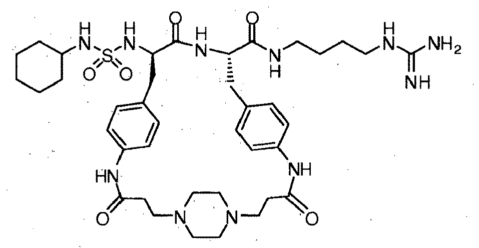 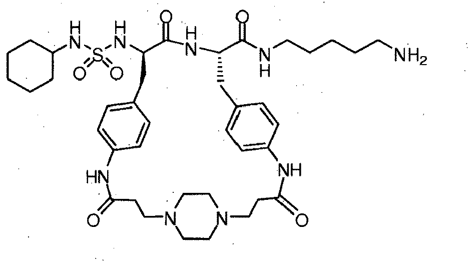 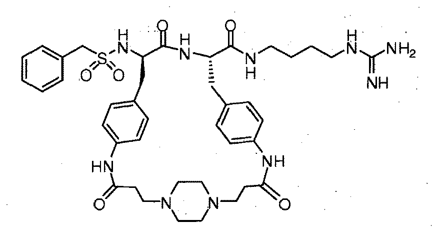 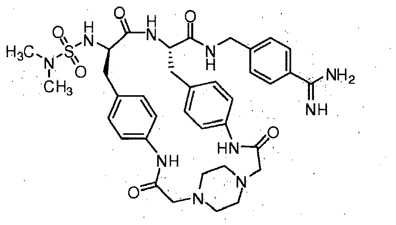 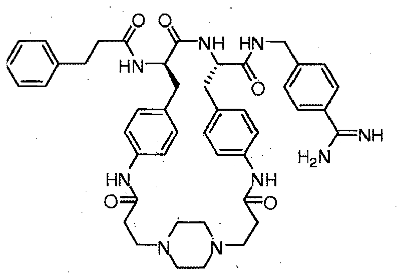 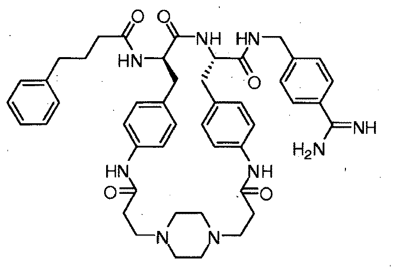 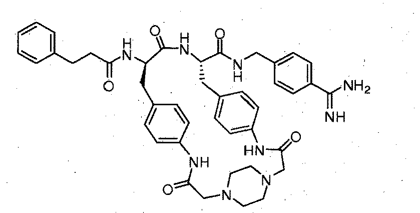 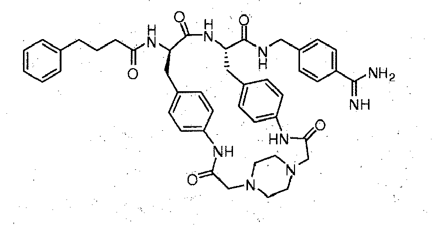 dans lequel les groupements basiques et éventuellement les groupements acides présents se présentent sous une forme de sel physiologiquement compatible.

8. Médicament contenant au moins un composé selon les revendications 1 à 7.

9. Médicament selon la revendication 8, **caractérisé en ce qu'**il est mis en oeuvre sous la forme d'un comprimé, d'une dragée, d'une capsule, d'un pellet, d'un suppositoire, d'une solution, en particulier une solution d'injection ou de perfusion, de gouttes pour les yeux, le nez ou les oreilles, d'un jus, d'une émulsion ou d'une suspension, d'un globule, d'un bâton, d'un aérosol, d'un spray d'aérosol, d'une poudre, d'une pâte, d'une crème ou d'un onguent, et on l'utilise de préférence une solution d'injection ou de perfusion.

10. Composés selon les revendications 1 à 7 et médicament selon les revendications 8 à 9, pour utilisation dans la réduction de la perte de sang dans le cas d'états hyper fibrinolytiques.

11. Composés selon les revendications 1 à 7 et médicament selon les revendications 8 à 9, permettant d'éviter les pertes de sang lors d'interventions chirurgicales, en particulier lors d'opérations du coeur et de transplantations d'organes, mais également pour alléger les fortes hémorragies menstruelles, pour empêcher ou modérer les saignements lors d'une extraction de dent ou les saignements des gencives, surtout chez les patients atteints d'hémophilie, mais également pour la thérapie de formes légères de l'hémophilie.

12. Utilisation des composés selon les revendications 1 à 7 et du médicament selon les revendications 8 et 9 comme moyen pour la fabrication d'une colle à base de fibrine.

13. Composés selon les revendications 1 à 7 et médicament selon les revendications 8 et 9 pour le traitement du cancer ou de la formation de métastases, pour le traitement de l'arthrite ou de la vitréorétinopathie proliférative.

14. Composés selon les revendications 1 à 7 et médicament selon les revendications 8 et 9, pour utilisation comme antidote dans les thérapies thrombolytiques avec de la plasmine ou des activateurs de plasmine, en particulier lors d'infarctus cardiaques, dans le cas où cela mène à des complications hémorragiques.

15. Utilisation des composés selon les revendications 1 à 7 pour des applications in vitro pour l'inhibition de la plasmine, y compris à des fins diagnostiques.
